# EUROPEAN PATENT APPLICATION

(11) **EP 4 023 757 A1**
(43) Date of publication of application: **06.07.2022**
(21) Application number: 20217528.7
(22) Date of filing: 29.12.2020
(51) Int. Cl.: C12N 15/80

(54) **METHODS FOR GENERATING SELECTION MARKER-FREE TRANSFORMANTS OF HETEROKARYOTIC ORGANISMS**

(71) Applicant: The Protein Brewery B.V., 4817 MV Breda (NL)
(72) Inventor: Parenicová, Lucie, 2497 ZJ Den Haag (NL); Reijngoud, Jos, 2405 VT Alphen aan den Rijn (NL)
(74) Representative: Nederlandsch Octrooibureau

(57) **Abstract**

The present invention relates to methods for generating selection marker gene-free recombinant strains. The methods can be applied to organisms having multinucleated cells, such as heterokaryotic cells, of which multinucleated fungal cells are preferred. In the method a multinucleated cell is co-transformed with at least two separate nucleic acid constructs, one construct comprising a selection marker and the other construct comprising a gene of interest. The method then employs a combination of selection, screening and segregation of multinucleated heterokaryotic cells into homokaryotic cells to select marker gene-free recombinant strains comprising the gene of interest.

## Description

### Field of the invention

The present invention relates to the field of molecular microbiology. In particular, the invention relates to methods and means for generating selection marker-free transformants of heterokaryotic organisms. Specifically, the invention relates to methods and means for generating selection marker-free transformants of heterokaryotic filamentous fungi.

### Background of the invention

Filamentous fungi, often those being employed in industry for production of enzymes or metabolites, are known to contain genetically distinct nuclei within the same cell (Roper et al., 2011; Strom et al., 2016). This characteristic, heterokaryosis, allows for a potent use of filamentous fungi such as *Penicillum chrysogenum, Acremonium chrysogenum, Aspergillus sojae, Aspergillus niger* and others in biotechnology for production of target compounds (metabolites and proteins), through high transcription and translation cellular capacity, generally better fitness of the microorganism (due to mutual complementation of allelic mutations) or creation of new genotypes (in case that fusion of compatible nuclei occurs). Heterokaryosis can also be employed in the identification of essential genes in A. *niger* via the generation of balanced heterokaryons in primary transformants (Mayer et al., 2007).

In the process of generating transformants, heterokaryosis is seen as unwanted and there is a strong desire to select for a single transformed nucleus with well-defined genomic features, such as the place of integration of the gene of interest (GOI) in the genome and the copy number of the gene of interest (EFSA Scientific Opinion, 2011 and 2018). The presence of non-identical, multinucleate cells can lead to genetic instability of production strain during fermentation process, causing process inconsistency and can eventually even lead to loss of the traits of the interest (the transgene and/or the favorable genotype).

Many of the filamentous fungi transformation protocols are based on transforming protoplasts (review by Ruiz-Diez, B. (2002)), which are multinuclear as they are derived from growing mycelium, in which the nuclei can migrate between the cells (Strom N. B. et al. (2016)). The protoplasts are transformed with a gene of interest (coding for instance for an enzyme) and a selection marker gene, which allows regeneration only of those protoplasts, whose nucleus has acquired the transforming DNA. In the process of selecting transformants, typically a selection medium is used, that is compatible with the type of the selection marker gene transformed. Nowadays, there is a broad pallet of selection markers that can be applied during genetic manipulation of filamentous fungi, that can be categorized into three groups: (i) antibiotic resistance markers, (ii) auxotrophic markers, and (iii) nutritional markers (see e.g. review by Meyer et al., 2011). The auxotrophic and nutritional group of selection markers is of special interest to industry for building fungal production strains, because it avoids using an antibiotic resistant marker and some of these markers allow for counter-selection of the marker gene (e.g. *amdS* on fluoroacetamide or *pyrG* on 5-fluoro-orotic acid). This results in marker-free transformants, which will give the final production strain a regulatory advantage, since it limits the presence of foreign DNA in the production organism.

The selection marker can be placed on one DNA fragment, together with a gene of interest (usually ratio 1:1), or can be co-transformed with a gene of interest on a different DNA fragment (ratio marker gene: gene of interest can vary). The latter approach can lead to genotypically different nuclei within one regenerating, transformed protoplast. Subsequently, these nuclei are sorted to predominantly single-nucleus spores in the process of sporulation of a transformant. In the next step, the transformants are purified generally using selection plates compatible with the transformed selection marker, on which the spores are plated. In this way, the purification of the transformants is driven by the selection marker and finally will lead to a homokaryon transformant carrying the selection marker and the gene of interest. In the case of co-transformation, not all purified transformants will be co-transformants, e.g. carrying next to the marker gene also the gene of interest. Besides the fungal protoplast transformation, similar effects can occur in other transformation methods that will target multinucleate cells, such as the *Agrobacterium* mediated transformation, electroporation of germinating spores, ballistic method or others (review by Ruiz-Diez, 2002).

The transformation of filamentous fungi has been intensively covered in for example US 4,885,249 (*arg*B-based selection), US 5,876,988 (amdS-based generation of selection-marker free recombinant strains) or US6,548,285 (*A*. *niger* and *P. chrysogenum amdS* selectable marker).

These patents, amongst others, addresses the difficulty to have a homologous selection marker for the fungal species, which should be transformed; the right genotype of the transforming strain, that would allow the use of the selection marker; the regulatory restrictions of use of a certain selection marker; the problem to generate a high copy number transformant etc.

There is however still a need for improved methods and means for generating selection marker-free recombinant organisms. It is an objection of the instant invention to provide for such methods and means.

### Summary of the invention

In a first aspect, the invention relates to a method for obtaining a selection marker gene-free recombinant strain. The method preferably comprising at least the following steps: a) co-transformation of a multinucleated cell with at least two separate nucleic acid constructs, wherein the first nucleic acid construct comprises an expression cassette for expression in the cell of a selection marker, and wherein a second nucleic acid construct comprises a gene of interest; b) selection of the multinucleated cell as obtained in a) for a transformed heterokaryotic cell expressing the selection marker; c) screening of the transformed heterokaryotic cell as obtained in b) for a heterokaryotic cell of which at least one nucleus comprises the gene of interest; d) segregating the heterokaryotic cell of which at least one nucleus comprises the gene of interest as obtained in c) into one or more homokaryotic cells; and, e) screening and/or selecting the one or more homokaryotic cells as obtained in d) for at least one cell that comprises the gene of interest and that lacks the selection marker, to obtain the selection marker gene-free recombinant strain. In the method of the invention, preferably, in step d) the heterokaryotic cell is segregated into one or more homokaryotic cells by sporulating the heterokaryotic cell. In the method of the invention, preferably, in step e) the one or more homokaryotic cells as obtained in d) are subjected to a differential selection regime that allows to distinguish on the basis of a phenotypic difference between a cell that lacks the selection marker and a cell that expresses the selection marker, wherein more preferably, the phenotypic difference is a difference in growth properties expressed under the differential selection regime, wherein most preferably, the differential selection regime is on a solid medium and a cell that lacks the selection marker is distinguished from a cell that expresses the selection marker on the basis of colony size or shape.

In one embodiment, the method of the invention is a method wherein the selection marker is a bidirectional marker and in step e) the one or more homokaryotic cells as obtained in d) are subjected to a counter-selection regime that selects against cells expressing the selection marker.

In one embodiment, the method of the invention is a method wherein the at least one cell that comprises the gene of interest and that lacks the selection marker, as obtained in step e), is subjected to a further round of segregation into one or more homokaryotic cells, preferably by sporulation, and optionally followed by at least one of screening and selection to identify a selection marker gene-free recombinant strain having the desired genotype.

In one embodiment, the method of the invention is a method wherein at least one of the first and second nucleic acid constructs are integrative nucleic acid constructs.

In one embodiment, the method of the invention is a method wherein the multinucleated cell to be co-transformed with the at least at least two nucleic acid constructs is a cell of a heterokaryotic organism.

In one embodiment, the method of the invention is a method wherein the multinucleated cell to be co-transformed with the at least at least two nucleic acid constructs is a cell of a filamentous fungus or a yeast.

In one embodiment, the method of the invention is a method wherein the filamentous fungus belongs to a genus selected from the genera including *Alternaria, Apophysomyces, Aspergillus, Cladosphialophora, Fonsecaea, Fusarium, Lichtheimia, Myceliophthora, Rhizopus, Rhizomucor, Trichoderma* and *Trichophyton,* or wherein the yeast belongs to a genus selected from the genera including *Candida, Hansenula, Kluyveromyces, Pichia, Saccharomyces, Schizosaccharomyces,* and *Yarrowia.*

In one embodiment, the method of the invention is a method wherein the filamentous fungus belongs a species selected from *Alternaria alternata, Apophysomyces variabilis, Aspergillus* spp., *Aspergillus fumigatus, Aspergillus flavus, Aspergillus oryzae, Aspergillus niger, Aspergillus awamori, Aspergillus nidulans, Aspergillus terreus, Cladosphialophora* spp., *Fonsecaea pedrosoi, Fusarium* spp., *Fusarium oxysporum, Fusarium solani, Lichtheimia* spp., *Lichtheimia corymbifera, Lichtheimia ramosa, Myceliophthora* spp., *Myceliophthora thermophila, Rhizopus* spp., *Rhizopus microsporus, Rhizomucor spp., Rhizomucor pusillus, Rhizomucor miehei, Trichoderma* spp., *Trichoderma reesei, Trichophyton* spp., *Trichophyton interdigitale,* and *Trichophyton rubrum,* of which the species *Aspergillus niger* is most preferred, or wherein the yeast belongs to a species selected from the species *Kluyveromyces lactis, Saccharomyces cerevisiae, Hansenula polymorpha, Yarrowia lipolytica* and *Pichia pastoris.*

In one embodiment, the method of the invention is a method wherein the selection marker encodes an acetamidase, wherein preferably the selection marker is an *Aspergillus amdS* gene.

In one embodiment, the method of the invention is a method wherein the gene of interest encodes a polypeptide of interest or a DNA or RNA molecule of interest.

In one embodiment, the method of the invention is a method wherein the polypeptide of interest is selected from the group consisting of: an antibody or antigen-binding fragment thereof, an antigen, a clotting factor, an animal-derived food-protein, an enzyme, a hormone or a hormone variant, a receptor or parts thereof, a regulatory protein, a structural protein, a reporter, or a transport protein, a protein involved in secretion process, a protein involved in folding process, a chaperone, a peptide amino acid transporter, a glycosylation factor, a transcription factor, a synthetic peptide or oligopeptide and an intracellular protein.

### Description of the figures

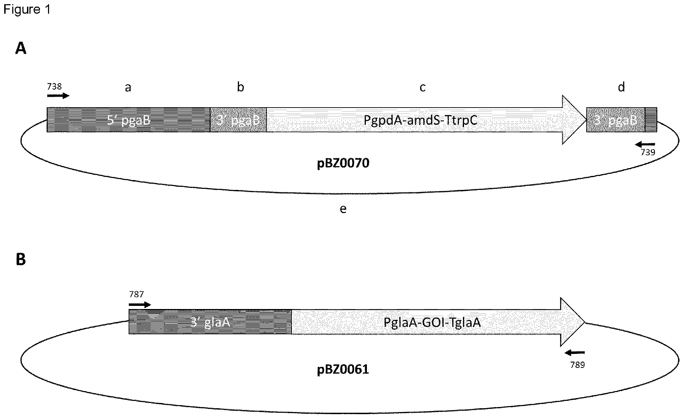

Figure 1 Overview of the plasmids used to PCR amplify the transformation constructs. The *amdS* selection cassette is flanked by sequences targeting at the *pgaB* locus (A), the GOI expression construct is targeted at the *glaA* locus (B).

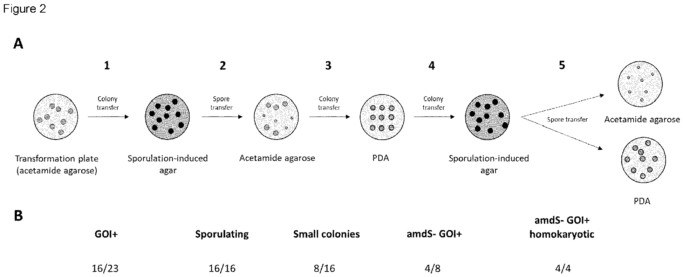

Figure 2 Overview of the segregant purification process (A) and the amount of correct colonies for each step in the process (B). In step 1 colonies are selected for the presence of the GOI using colony PCR. In step 2 sporulation is induced to force segregation of nuclei into spores. In step 3 segregants are selected with moderate growth on acetamide (small colonies). In step 4 biomass of small colonies is regenerated and segregants are selected for being *amdS-* and GOI+ using colony PCR. Finally, in step 5 the remaining segregants are tested for being homokaryon by analysing ≥8 single spores isolates for the presence of *amdS* and the GOI. Phenotypically the presence of the *amdS* gene is analyzed by plating spores on acetamide medium. Genotypically the presence of *amdS* and ova is analyzed using colony PCR.

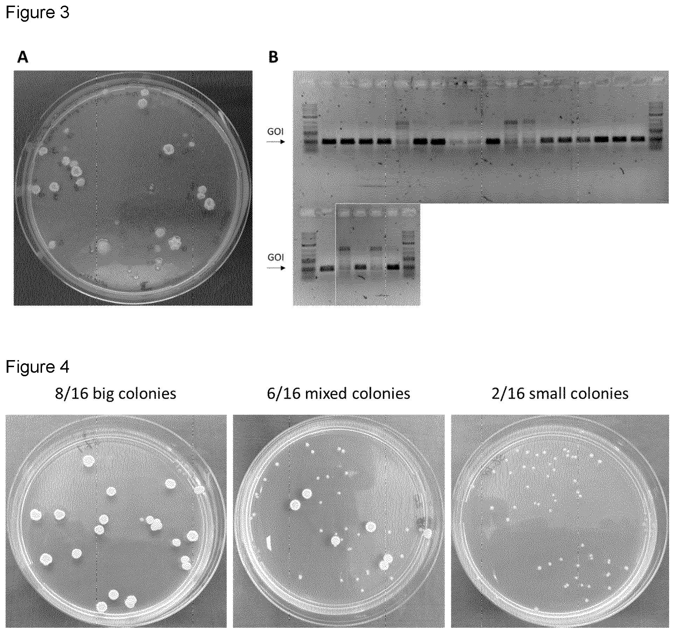

Figure 3 In step 1, the transformants are genotypically analyzed. 23 Transformants were growing on acetamide medium (A). Colony PCR showed the presence of the GOI in 16 out of 23 (16/23) transformants (B).
Figure 4 In step 3, 10-100 spores of the transformants are phenotypically analyzed for growth on acetamide medium. Small colonies are *amdS-* segregants, big colonies are *amdS*+ segregants. Three different groups could be classified: transformants with only big colonies (left), transformants with a mix of big and small colonies (middle) and transformants with only small colonies (right). For every transformant having at least 1 small colony, 4 colonies were selected to continue with.

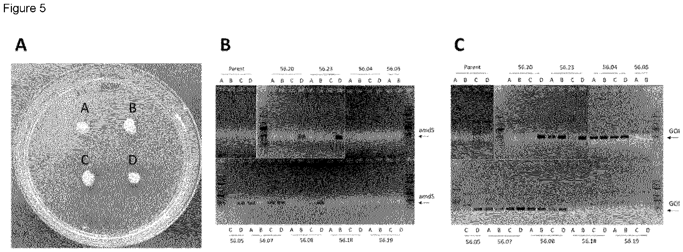

Figure 5 In step 4, the four selected colonies were grown on rich PDA medium (A) to allow genotypic analysis with colony PCR. Presence or absence of *amdS* (B) and of the GOI (C) were investigated. Four transformants showed at least one segregant having the desired *amdS-* GOI+ genotype.

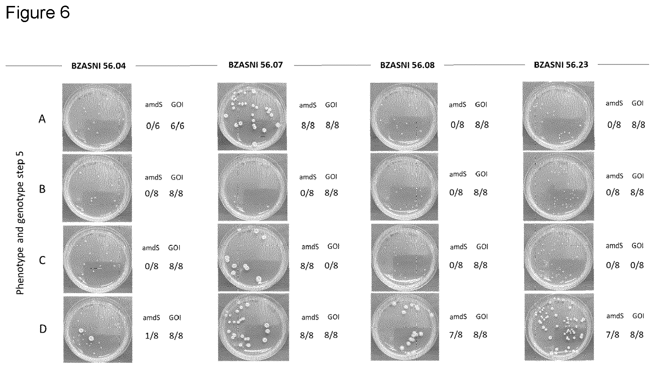

Figure 6 In step 5, spores of the four transformants having at least one segregant with the *amdS-*GOI+ genotype were plated on acetamide medium to phenotypically verify their *amdS-* genotype. All genotypes analyzed in step 4 match with their phenotype in step 5, except for 56.04 D (which supposes to be *amdS-* but still possesses two bigger colonies on acetamide). Similarly, spores were plated on PDA medium to genotypically verify their *amdS-* and GOI+ genotype with colony PCR. 6-8 colonies per segregant were investigated (indicated next to image). Again, the genotypes in step 4 match their genotype in step 5, except for the segregants originating from bigger colonies (in step 3: #D). Segregants A-C all appear to be of homokaryon nature, albeit segregants originating from bigger colonies seem to be heterokaryotic.

### Description of the invention

### Definitions

Unless defined otherwise, technical and scientific terms used herein have the same meaning as commonly understood by one of ordinary skill in the art to which this disclosure belongs. One skilled in the art will recognize many methods and materials similar or equivalent to those described herein, which could be used in the practice of the present invention. Indeed, the present invention is in no way limited to the method.

For purposes of the present invention, the following terms are defined below.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

As used herein, the term "and/or" indicates that one or more of the stated cases may occur, alone or in combination with at least one of the stated cases, up to with all of the stated cases.

As used herein, with "At least" a particular value means that particular value or more. For example, "at least 2" is understood to be the same as "2 or more" i.e., 2, 3, 4, 5, 6, 7, 8, 9, 10, 11, 12, 13, 14, 15, ... ,etc.

The word "about" or "approximately" when used in association with a numerical value (e.g. about 10) preferably means that the value may be the given value (of 10) more or less 0.1% of the value.

The terms "homology", "sequence identity" and the like are used interchangeably herein. Sequence identity is herein defined as a relationship between two or more amino acid (polypeptide or protein) sequences or two or more nucleic acid (polynucleotide) sequences, as determined by comparing the sequences. In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods.

"Sequence identity" and "sequence similarity" can be determined by alignment of two peptide or two nucleotide sequences using global or local alignment algorithms, depending on the length of the two sequences. Sequences of similar lengths are preferably aligned using a global alignment algorithms (e.g. Needleman Wunsch) which aligns the sequences optimally over the entire length, while sequences of substantially different lengths are preferably aligned using a local alignment algorithm (e.g. Smith Waterman). Sequences may then be referred to as "substantially identical" or "essentially similar" when they (when optimally aligned by for example the programs GAP or BESTFIT using default parameters) share at least a certain minimal percentage of sequence identity (as defined below). GAP uses the Needleman and Wunsch global alignment algorithm to align two sequences over their entire length (full length), maximizing the number of matches and minimizing the number of gaps. A global alignment is suitably used to determine sequence identity when the two sequences have similar lengths. Generally, the GAP default parameters are used, with a gap creation penalty = 50 (polynucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). Sequence alignments and scores for percentage sequence identity may be determined using computer programs, such as the GCG Wisconsin Package, Version 10.3, available from Accelrys Inc., 9685 Scranton Road, San Diego, CA 92121-3752 USA, or using open source software, such as the program "needle" (using the global Needleman Wunsch algorithm) or "water" (using the local Smith Waterman algorithm) in EmbossWIN version 2.10.0, using the same parameters as for GAP above, or using the default settings (both for 'needle' and for 'water' and both for protein and for DNA alignments, the default Gap opening penalty is 10.0 and the default gap extension penalty is 0.5; default scoring matrices are Blosum62 for proteins and DNAFull for DNA). When sequences have a substantially different overall lengths, local alignments, such as those using the Smith Waterman algorithm, are preferred.

Alternatively percentage similarity or identity may be determined by searching against public databases, using algorithms such as FASTA, BLAST, etc. Thus, the nucleic acid and protein sequences of the present invention can further be used as a "query sequence" to perform a search against public databases to, for example, identify other family members or related sequences. Such searches can be performed using the BLASTn and BLASTx programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to the nucleic acid molecules of the invention. BLAST protein searches can be performed with the BLASTx program, score = 50, wordlength = 3 to obtain amino acid sequences homologous to protein molecules of the invention. To obtain gapped alignments for comparison purposes, Gapped BLAST can be utilized as described in Altschul et al., (1997) Nucleic Acids Res. 25(17): 3389-3402. When utilizing BLAST and Gapped BLAST programs, the default parameters of the respective programs (e.g., BLASTx and BLASTn) can be used. See the homepage of the National Center for Biotechnology Information at http://www.ncbi.nlm.nih.gov/.

As used herein, the term "selectively hybridizing", "hybridizes selectively" and similar terms are intended to describe conditions for hybridization and washing under which nucleotide sequences at least 66%, at least 70%, at least 75%, at least 80%, more preferably at least 85%, even more preferably at least 90%, preferably at least 95%, more preferably at least 98% or more preferably at least 99% homologous to each other typically remain hybridized to each other. That is to say, such hybridizing sequences may share at least 45%, at least 50%, at least 55%, at least 60%, at least 65, at least 70%, at least 75%, at least 80%, more preferably at least 85%, even more preferably at least 90%, more preferably at least 95%, more preferably at least 98% or more preferably at least 99% sequence identity.

A preferred, non-limiting example of such hybridization conditions is hybridization in 6X sodium chloride/sodium citrate (SSC) at about 45°C, followed by one or more washes in 1 X SSC, 0.1% SDS at about 50°C, preferably at about 55°C, preferably at about 60°C and even more preferably at about 65°C.

Highly stringent conditions include, for example, hybridization at about 68°C in 5x SSC/5x Denhardt's solution / 1.0% SDS and washing in 0.2x SSC/0.1% SDS at room temperature. Alternatively, washing may be performed at 42°C.

The skilled artisan will know which conditions to apply for stringent and highly stringent hybridization conditions. Additional guidance regarding such conditions is readily available in the art, for example, in Sambrook et al., 1989, Molecular Cloning, A Laboratory Manual, Cold Spring Harbor Press, N.Y.; and Ausubel et al. (eds.), Sambrook and Russell (2001) "Molecular Cloning: A Laboratory Manual (3rd edition), Cold Spring Harbor Laboratory, Cold Spring Harbor Laboratory Press, New York 1995, Current Protocols in Molecular Biology, (John Wiley & Sons, N.Y.).

Of course, a polynucleotide which hybridizes only to a poly A sequence (such as the 3' terminal poly(A) tract of mRNAs), or to a complementary stretch of T (or U) resides, would not be included in a polynucleotide of the invention used to specifically hybridize to a portion of a nucleic acid of the invention, since such a polynucleotide would hybridize to any nucleic acid molecule containing a poly (A) stretch or the complement thereof (e.g., practically any double-stranded cDNA clone).

A "nucleic acid construct" or "nucleic acid vector" is herein understood to mean a man-made nucleic acid molecule resulting from the use of recombinant DNA technology. The term "nucleic acid construct" therefore does not include naturally occurring nucleic acid molecules although a nucleic acid construct may comprise (parts of) naturally occurring nucleic acid molecules. The terms "expression vector" or "expression construct" refer to nucleotide sequences that are capable of effecting expression of a gene in host cells or host organisms compatible with such sequences. These expression vectors typically include at least suitable transcription regulatory sequences and optionally, 3' transcription termination signals. Additional factors necessary or helpful in effecting expression may also be present, such as expression enhancer elements. The expression vector will be introduced into a suitable host cell and be able to effect expression of the coding sequence in an in vitro cell culture of the host cell. The expression vector will be suitable for replication in the host cell or organism of the invention.

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active in most tissues under most physiological and developmental conditions. An "inducible" promoter is a promoter that is physiologically or developmentally regulated, e.g. by the application of a chemical inducer. An inducible promoter may also be present but not induced.

The term "selectable marker" is a term familiar to one of ordinary skill in the art and is used herein to describe any genetic entity which, when expressed, can be used to select for a cell or cells containing the selectable marker. The term "reporter" may be used interchangeably with marker, although it is mainly used to refer to visible markers, such as green fluorescent protein (GFP). Selectable markers may be dominant or recessive or bidirectional.

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame.

The terms "protein" or "polypeptide" are used interchangeably and refer to molecules consisting of a chain of amino acids, without reference to a specific mode of action, size, 3-dimensional structure or origin.

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region, exons, introns and a 3'-nontranslated sequence (3'-end) e.g. comprising a polyadenylation- and/or transcription termination site.

"Expression of a gene" refers to the process wherein a DNA region which is operably linked to appropriate regulatory regions, particularly a promoter, is transcribed into an RNA, which is biologically active, i.e. which is capable of being translated into a biologically active protein or peptide.

The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain. If homologous to a host cell, a nucleic acid sequence encoding a polypeptide will typically (but not necessarily) be operably linked to another (heterologous) promoter sequence and, if applicable, another (heterologous) secretory signal sequence and/or terminator sequence than in its natural environment. It is understood that the regulatory sequences, signal sequences, terminator sequences, etc. may also be homologous to the host cell. In this context, the use of only "homologous" sequence elements allows the construction of "self-cloned" genetically modified organisms (GMO's) (self-cloning is defined herein as in European Directive 98/81/EC Annex II). When used to indicate the relatedness of two nucleic acid sequences the term "homologous" means that one single-stranded nucleic acid sequence may hybridize to a complementary single-stranded nucleic acid sequence. The degree of hybridization may depend on a number of factors including the amount of identity between the sequences and the hybridization conditions such as temperature and salt concentration as discussed earlier herein.

The terms "heterologous" and "exogenous" when used with respect to a nucleic acid (DNA or RNA) or protein refers to a nucleic acid or protein that does not occur naturally as part of the organism, cell, genome or DNA or RNA sequence in which it is present, or that is found in a cell or location or locations in the genome or DNA or RNA sequence that differ from that in which it is found in nature. Heterologous and exogenous nucleic acids or proteins are not endogenous to the cell into which it is introduced, but have been obtained from another cell or synthetically or recombinantly produced. Generally, though not necessarily, such nucleic acids encode proteins, i.e. exogenous proteins, that are not normally produced by the cell in which the DNA is transcribed or expressed. Similarly exogenous RNA encodes for proteins not normally expressed in the cell in which the exogenous RNA is present. Heterologous/exogenous nucleic acids and proteins may also be referred to as foreign nucleic acids or proteins. Any nucleic acid or protein that one of skill in the art would recognize as foreign to the cell in which it is expressed is herein encompassed by the term heterologous or exogenous nucleic acid or protein. The terms heterologous and exogenous also apply to non-natural combinations of nucleic acid or amino acid sequences, i.e. combinations where at least two of the combined sequences are foreign with respect to each other. The terms heterologous and exogenous specifically also apply to non-naturally occurring modified versions of otherwise endogenous nucleic acids or proteins.

The "specific activity" of an enzyme is herein understood to mean the amount of activity of a particular enzyme per amount of total host cell protein, usually expressed in units of enzyme activity per mg total host cell protein. In the context of the present invention, the specific activity of a particular enzyme may be increased or decreased as compared to the specific activity of that enzyme in an (otherwise identical) wild type host cell.

The term "fungal", when referring to a protein or nucleic acid molecule thus means a protein or nucleic acid whose amino acid or nucleotide sequence, respectively, naturally occurs in a fungus.

### Detailed description of the invention

The present invention provides methods for the transformation of heterokaryotic organisms, such as filamentous fungi, and for the subsequent purification of genotypically homogeneous clones, which allows for faster isolation of marker-free transformants. The methods of the invention exploit the multinuclear character of heterokaryotic cells, by using at least two nucleic acid constructs in co-transformation - one construct carrying a selection marker and the others carrying one or more a genes of interest.

In some embodiments the methods of the invention are not driven by a selection of transformants based on the presence of the marker gene, but rather by the selection of transformants based on the presence of the gene of interest. In this way, the isolation of transformants containing only the selection marker is substantially minimized or almost eliminated.

In some embodiments, the methods of the invention do not include a counter selection step selecting for the loss of the marker gene cassette, which also prevents a possible loss of one or more or all copies of the gene of interest.

In some embodiment, the methods of the invention makes use of phenotypic differences between the transformants carrying the marker gene and the transformants, that do not possess the marker gene, when grown on a selection medium. These phenotypic differences, e.g. differential growth rate causing differences in the sizes of colonies comprising the marker gene as compared to colonies lacking the marker gene, can e.g. be caused by a residual enzymatic background activity (e.g. allowing some growth on acetamide as sole C- or N-source) or a minimal level of antibiotic resistance to lower concentrations of the antibiotic in an untransformed strain. These phenotypic differences thus allow to distinguish between transformants that do and do not comprise the selection marker gene and thereby allow to identify and isolate selection marker-free transformants. Thus, in one embodiment, the method of the invention uses a mildly selective medium that allows some growth of cells lacking the selection marker and at the same time offers a growth advantage to cells comprising the selection marker.

In one embodiment, the method of the invention is based on transfer of the primary transformants, preferably transfer of the fragmented mycelium of the primary transformants, (e.g. recovered transformed cells/protoplast on a selective medium) to a non-selective medium, which induces sporulation of the transformants.

In some embodiments, the method of the invention employs nucleic acid constructs that integrate into the host cell's genome. In other embodiments, the method of the invention employs episomal nucleic acid constructs that are capable of autonomous replication in the host cell.

In some embodiments, the method of the invention is based on a strain that is incapable of sporulating on transformation media, but this approach might also be functional on sporulating strains (although you might need to find repressing sporulation medium).

In a first aspect therefore, the invention pertains to a method for obtaining a selection marker gene-free recombinant strain. The method preferably comprises at least the steps of: a) co-transformation of a multinucleated cell with at least two separate nucleic acid constructs, wherein the first nucleic acid construct comprises an expression cassette for expression in the cells of a selection marker, and wherein a second nucleic acid construct comprises a gene of interest; b) selection of the multinucleated cell as obtained in a) for a transformed heterokaryotic cell expressing the selection marker; c) screening of the transformed heterokaryotic cell as obtained in b) for a heterokaryotic cell of which at least one nucleus comprises the gene of interest; d) segregating the heterokaryotic cell of which at least one nucleus comprises the gene of interest as obtained in c) into a plurality of homokaryotic cells; and, e) screening and/or selecting the plurality of homokaryotic cells as obtained in d) for at least one cell that comprises the gene of interest and that lacks the selection marker, to obtain the selection marker gene-free recombinant strain.

In one embodiment of the method, the multinucleated cell to be co-transformed with the at least two nucleic acid constructs is understood to be cell that comprise more than one nucleus per cell. For all practical purposes, a multinucleated cell to be co-transformed can be a plurality of the multinucleated cell, such as a population or a culture of multinucleated cells. A multinucleated cell to be co-transformed can be a homokaryotic cell comprising multiple genotypically identical nuclei per cell, or a multinucleated cell to be co-transformed can be a heterokaryotic cell, i.e. comprising at least two genotypically different nuclei per cell. In one embodiment, a multinucleated cell to be co-transformed is a cell of a heterokaryotic organism. The heterokaryotic organism can be a fungus, a ciliate protozoan, such as *Tetrahymena* or a slime mould, such as *Physarum* or *Dictyostelium.*

In a preferred embodiment, a multinucleated cell to be co-transformed is a cell of a species that belong to the "fungi", which are herein defined as eukaryotic microorganisms, which include all species of the subdivision Eumycotina (Alexopoulos, C. J., 1962, In: Introductory Mycology, John Wiley & Sons, Inc., New York). The term fungus thus includes both filamentous fungi and yeast and it is understood herein that a multinucleated cell to be co-transformed can be a cell of a yeast or a cell of a filamentous fungus. Fungal genera and species for use in the methods of the invention are further specified herein below.

In step a) of the method of the invention, a multinucleated cell is co-transformed with the at least two separate nucleic acid constructs. Methods for transformation of one or more nucleic acid constructs are generally known in the art and include methods such as protoplast or spheroplast transformation, electroporation, sonoporation, biolistics as well as chemicals such as alkali cations (e.g. caesium or lithium), calcium, and/or polyethylene glycol. Depending on the species to which the cell to be transformed belongs the skilled person knows which transformation protocol is to be applied. Transformation of fungi may involve a process consisting of protoplast formation, transformation of the protoplasts, and regeneration of the cell wall in a manner known per se. Suitable procedures for transformation of *Aspergillus cells* are described in EP 238 023 and Yelton et al., 1984, Proceedings of the National Academy of Sciences USA 81 : 1470- 1474. Suitable procedures for transformation of *Aspergillus* and other filamentous fungal host cells using *Agrobacterium tumefaciens* are described in e.g. Nat Biotechnol. 1998 Sep;16(9):839-42. Erratum in: Nat Biotechnol 1998 Nov;16(11):1074. *Agrobacterium* tumefaciens-mediated transformation of filamentous fungi, de Groot MJ, Bundock P, Hooykaas PJ, Beijersbergen AG. Unilever Research Laboratory Vlaardingen, The Netherlands. A suitable method of transforming *Fusarium* species is described by Malardier et al., 1989, Gene 78: 147156 or in WO 96/00787. Other method can be applied such as a method using biolistic transformation as described in: Biolistic transformation of the obligate plant pathogenic fungus, *Erysiphe graminis* f.sp. *hordei.* Christiansen SK, Knudsen S, Giese H. Curr Genet. 1995 Dec; 29(1):100-2. Yeast may be transformed using the procedures described by Becker and Guarente, In Abelson, J. N. and Simon, M. I., editors, Guide to Yeast Genetics and Molecular Biology, Methods in Enzymology, Volume 194, pp 182-187, Academic Press, Inc., New York; Ito et al., 1983, Journal of Bacteriology 153: 163; and Hinnen et al., 1978, Proceedings of the National Academy of Sciences USA 75: 1920. For is co-transformation with the at least two separate nucleic acid constructs, the at least two nucleic acid constructs are preferably mixed in a desired ratio prior to being subject to a suitable transformation method as described above.

In step a) of the method of the invention, a multinucleated cell is co-transformed with at least two separate nucleic acid constructs, which is understood to mean that the first and second nucleic acid constructs are two separate nucleic acid molecules that are not physically linked such that each nucleic acid construct can "transform" a different nucleus in the multinucleated cell.

In one embodiment of the method of the invention, the at least two separate nucleic acid constructs (that are used co-transform a multinucleated cell) include at least a first nucleic acid construct comprising an expression cassette for expression in the cells of a selection marker, and a second nucleic acid construct comprises at least one gene of interest.

Means and methods for constructing the expression vectors and cassettes of the present invention are well known to one skilled in the art (see, e.g., Sambrook and Russell, *supra*; and Ausubel et al., Current Protocols in Molecular Biology, Wiley InterScience, NY, 1995).

Thus, in one embodiment, the first nucleic acid construct comprises an expression cassette for expression in the cells of a selection marker. Expression of the selection marker, after successful transformation of at least a fraction of the multinucleated cells, will permit easy selection of transformed heterokaryotic cells. Using the method of co-transformation, one vector may contain the selectable marker whereas another vector may contain the polynucleotide of interest or the nucleic acid construct of interest; the vectors can be simultaneously used for transformation of the multinucleated host cell. A selection marker can be a gene, the product of which provides for biocide, antibiotic or viral resistance, resistance to heavy metals, prototrophy to auxotrophs, and the like. Selection markers for use in the methods of the invention are further specified herein below.

In one embodiment, the second nucleic acid construct comprises a polynucleotide or gene of interest. The gene of interest can be any polynucleotide of interest. The polynucleotide of interest can be from any prokaryotic, eukaryotic, vertebrate, avian, mammalian, human, plant or other source. The polynucleotide of interest can be heterologous to the host cell or can be homologous to the host cell, resulting in a recombinant host cell being a self-clone. The polynucleotide of interest can encode a polypeptide of interest or a nucleic acid of interest such as a DNA or RNA molecule of interest. Polypeptides of interest for use in the methods of the invention are further specified herein below.

In one embodiment, the second nucleic acid construct comprises more than one gene of interest and/or the second nucleic acid construct comprises more than one expression cassettes comprising a gene of interest. In some embodiments, each expression cassette comprises a different gene of interest. In some embodiment, the first nucleic acid construct with the selection marker is co-transformed with a second and further nucleic acid constructs, each comprising a (different) gene of interest.

In some embodiments, the method of the invention employs nucleic acid constructs that integrate into the host cell's genome. In other embodiments, the method of the invention employs episomal nucleic acid constructs that are capable of autonomous replication in the host cell, such as the AMA1 -sequence (see e.g. Aleksenko and Clutterbuck (1997), Fungal Genet. Biol. 21 : 373-397) for *Aspergilli* or 2µ-based plasmids and similar for yeast. In a preferred embodiment, at least one of the first and second nucleic acid constructs are integrative constructs that are capable of integrating into the host cell's genome. Such integrative nucleic acid constructs preferably lack on origin of replication for the host cell. More preferably, an integrative nucleic acid construct comprises a DNA fragment that is homologous to a DNA sequence in a predetermined target locus in the genome of the host cell for targeting the integration of the cloning vectorto this predetermined locus. In order to promote targeted integration, the cloning vector is preferably linearized prior to transformation of the host cell. Linearization is preferably performed such that at least one but preferably either end of the cloning vector is flanked by sequences homologous to the target locus. The length of the homologous sequences flanking the target locus is preferably at least 30bp, preferably at least 50 bp, preferably at least 0.1 kb, preferably at least 0.2kb, preferably at least 0.5 kb, more preferably at least 1 kb, even more preferably at least 2 kb, most preferably at least 3 kb.

In one embodiment, in step b) of the method of the invention, the multinucleated cell as obtained in a) is subjected to a selection regime that favors a cell that expresses the selection marker. These selective conditions are chosen so as remove any untransformed cells (and cells that have not been transformed with the selection marker construct) and enrich for a fraction of the multinucleated cells co-transformed in step a) of the method that express the selection marker. The skilled person is well aware of how to apply such selective conditions, depending on the selection marker used and the host cell in question. In one embodiment, the selection in step b) is performed on a solid selection medium (e.g. an agar-based medium) so that a single colony isolates of each transformed cell is obtained. In an alternative embodiment this can be performed in liquid medium by limiting dilution. A multinucleated cell that is selected in step b) of the method of the invention will comprise at least one nucleus comprising the first nucleic acid construct, from which the selection marker is expressed. Other nuclei in that same cell may or may not comprise the co-transformed second nucleic acid construct. Hence, a cell selected in step b) of the method is a transformed heterokaryotic cell expressing the selection marker.

In some embodiments, in step b) of the method of the invention the transformed multinucleated cell as obtained in a) is selected under conditions that avoid or prevent sporulation of the transformed multinucleated cell. In another embodiment, the transformed multinucleated cell is of a strain that is incapable of sporulating under conditions applied for the selection of the transformed cell.

In one embodiment, in step c) of the method of the invention, the transformed heterokaryotic cell as obtained in step b) of the method is screened for a heterokaryotic cell of which at least one nucleus comprises the gene of interest. Screening of cells for the presence of the gene of interest can be performed in any manner known to the skilled person per se. Screening of cells for the presence of the gene of interest can be performed by genotypic and/or by phenotypic analysis. In one embodiment the cells are genotypically screened for the presence of the gene of interest, by amplification techniques such as PCR (e.g. colony PCR), by hybridization, such as colony hybridization and/or by sequencing. In another embodiment, if the gene of interest is expressed, it may be possible to screen for a cell of which at least one nucleus comprises the gene of interest on the basis of the phenotype of the cell. Cells can be screened for the presence of the gene of interest phenotypically by any means known to the skilled person per se. For example, in one embodiment, the presence of the product expressed from the gene of interest can be detected by immunological means, e.g. using antibodies against the product. In another embodiment, the gene of interest encodes an enzyme, the presence of which can be detected by assaying for the enzyme's activity, or increased (specific) activity, if the host cell already expressed the enzyme. The screening in step b) of the method of the invention thus identifies a cell comprising at least one nucleus expression the selection marker (due to the selection in step b), and comprising at least one nucleus comprising the gene of interest (due to the screening in step c). Hence, a cell selected in step c) of the method is a heterokaryotic cell of which at least one nucleus comprises the gene of interest (and expressing the selection marker).

Next, in step d) of the method of the invention, a heterokaryotic cell obtained in the foregoing steps is segregated into a homokaryotic cells. In one embodiment, the heterokaryotic cell is segregated into a plurality of homokaryotic cells. Segregation in the context of the method of the invention is not to be understood as the segregation of alleles (from a given genome). Rather Segregation in the context of the method of the invention is to be understood as the segregation of the different nuclei that are present in given multinucleated/heterokaryotic cell into at least one separate homokaryotic cell carrying a nucleus that is genotypically identical to only one of the different nuclei that were present in the multinucleated/heterokaryotic cell prior to segregation.

In one embodiment of the method of the invention, a heterokaryotic cell is segregated into one or more homokaryotic cells by sporulating the heterokaryotic cell. In some embodiment, a fungal heterokaryotic cell is segregated into one or more homokaryotic cells by sporulating the fungal cell. Methods for inducing a heterokaryotic cell, e.g. a fungal heterokaryotic cell, to sporulate are well known in the art per se. For example, sporulation is generally known to occur when growth rate is reduced and is hampered under conditions that favor rapid (mycelial) growth. Starvation or nutritional depletion often stimulates sporulation. Some typical low nutrient media include water agar media, half- or 1/4-strength PDA (Masangkay et al. 2000, Biocontrol Sci Technol. 10(4):385-397), and synthetic nutrient-poor agar medium (Nirenberg 1976 Mitt Biol Bundesanst Land-Forstwirtsch (Berlin-Dahlem). 169:1-117). In one embodiment, the one or more segregated/sporulated homokaryotic cells/spores are individually grown to obtain a genetically uniform strain or culture for each of the one or more of the segregated/sporulated homokaryotic cells/spores.

A homokaryotic cell as obtained in step d) of the method of the invention, can be one of three type of cells: i) a cell comprising the first nucleic acid construct, comprising an expression cassette for the selection marker (and not comprising the second nucleic acid construct comprising a gene of interest); ii) a cell comprising the second nucleic acid construct with a gene of interest (and not comprising the first nucleic acid construct with the selection marker); and iii) a cell comprising both the first and second nucleic acid constructs. In one embodiment, the method of the invention concerns the provision of a selection marker gene-free recombinant strain and therefore the second type of cell needs to be distinguished from the first and third types of cells, both of which contain the selection marker. In one embodiment, at least one of screening and selection is applied on the one or more homokaryotic cells as obtained in d), to obtain at least one cell that comprises the gene of interest and that lacks the selection marker, thereby providing a selection marker gene-free recombinant strain.

In one embodiment of the method of the invention, the one or more homokaryotic cells as obtained in d) are subjected (in step e)) to a differential selection regime that allows to distinguish between a cell that lacks the selection marker and a cell that contains the selection marker on the bases of a phenotypic difference. In one embodiment, the phenotypic difference is a difference in growth properties expressed under the differential selection regime. The differential selection regime preferably allows to distinguish between a cell that lacks the selection marker and a cell that contains the selection marker on the bases of a difference in growth properties the two types of cells under the applied differential selection regime such as a difference in growth rate or difference in amount of growth (e.g. number of divisions).

In one embodiment the differential selection regime is a medium with a nutrient composition that allows only a limited amount or limited rate of growth of cells lacking the selection marker while supporting a higher amount or higher rate of growth of cells expressing the selection marker. For example, in one embodiment, the selection marker encodes an acetamidase and the differential selection regime is a minimal medium with acetamide as sole N- and/or C-source. As in practice many fungi have some low background acetamidase activity level, a cell without the acetamidase will show limited and/or slow growth on a medium with acetamide as sole N- and/or C-source, which cell can be distinguished from the faster growing cell comprising the first nucleic acid construct from which the acetamidase selection marker is expressed at higher level. In another embodiment, the selection marker encodes an auxotrophic marker and the differential selection regime is a (minimal) medium comprising only a limited amount of the nutrient for which a cell lacking the marker is auxotroph, wherein growth of the cell lacking the marker stops once the nutrient is depleted, which can be distinguished from the faster and/or continued growth of a cell expressing the auxotrophic marker. In yet another embodiment, the selection marker encodes an resistance, e.g. against an antibiotic or other toxic compound (e.g. heavy metal), and the differential selection regime is a medium comprising a concentration of the antibiotic or toxic compound that still allows growth but limits the growth rate of a cell lacking the marker, which can be distinguished from the faster growth rate of a cell expressing the resistance marker.

In one embodiment, differential selection regime is on a solid medium, such as an agar-based medium, and a cell that lacks the selection marker is distinguished from a cell that expresses the selection marker on the basis of colony size and/or colony shape.

In a further embodiment, wherein the selection marker is a bidirectional marker (see below) and in step e) the one or more homokaryotic cells as obtained in d) are subjected to a counter-selection regime that selects against cells expressing the selection marker. In a preferred embodiment of the method of the invention, a counter-selection step is avoided as it may also lead to undesirable loss of copies of the gene of interest. That being said, counter-selection is certainly not excluded from the method of the invention as it can provide a convenient way of performing step e) of the method by selecting against homokaryotic cells expressing the selection marker and thereby allowing growth of only homokaryotic cells that comprise the gene of interest and that lack the selection marker.

In an alternative embodiment, a combination of screening and selection is applied in step e) to the one or more homokaryotic cells as obtained in d) to obtain at least one cell that comprises the gene of interest and that lacks the selection marker. In one such embodiment, the one or more homokaryotic cells as obtained in d) can be tested (in parallel) for growth under selective and non-selective conditions, e.g. by replica plating, to identify cells that lack the selection marker by their ability to grow only under non-selective condition. Thus identified cells can then further be screened for the presence of the gene of interest by methods and means as outlined above for step c) of the method. This will confirm that the thus identified cells, lacking the selection marker, indeed comprise the gene of interest.

In one embodiment, the screening and/or selection applied in step e) to the one or more homokaryotic cells as obtained in d) to obtain at least one cell that comprises the gene of interest and that lacks the selection marker includes an analysis of the genotype of the at least one cell in order to identify a cell having a desired genotype. In the context of the invention, a desired genotype will at least include the absence of the selection marker used in the co-transformation but may also include one or more of the genotypic characteristics such as copy number of the gene of interest, the genomic site(s) at which the gene of interest is integrated, whether desired gene replacements have taken place, etc..

In one embodiment of the invention, the at least one cell that comprises the gene of interest and that lacks the selection marker, as obtained in step e), can be subjected to a further round of segregation into one or more homokaryotic cells. In some embodiments, the (repeated) segregation of the at least one cell obtained in step e) is by sporulation the at least one cell, e.g. using sporulation methods described above. In one embodiment, the one or more re-segregated cells thus obtained are subjected to at least one of screening and selection to identify a selection marker gene-free recombinant strain having the desired genotype.

Thus, in one embodiment, the multinucleated cell to be co-transformed with the at least two nucleic acid constructs is a fungus, such as a filamentous fungus or a yeast as detailed herein below. "Filamentous fungi" are herein defined as eukaryotic microorganisms that include all filamentous forms of the subdivision Eumycotina and Oomycota (as defined by Hawksworth et al., In, Ainsworth and Bisby's Dictionary of The Fungi, 8th edition, 1995, CAB International, University Press, Cambridge, UK). The filamentous fungi are characterized by a mycelial wall composed of chitin, cellulose, glucan, chitosan, mannan, and other complex polysaccharides. Vegetative growth is by hyphal elongation and carbon catabolism is obligately aerobic. A preferred filamentous fungal cell that can be used in the method of the invention is a cell the belongs to a genus selected from the genera including, but are not limited to, *Alternaria, Apophysomyces, Aspergillus, Cladosphialophora, Fonsecaea, Fusarium, Lichtheimia, Myceliophthora, Rhizopus, Rhizomucor, Trichoderma* and *Trichophyton.* More preferably, a filamentous fungal host cell belong to a species selected from *Alternaria alternata, Apophysomyces variabilis, Aspergillus* spp., *Aspergillus fumigatus, Aspergillus flavus, Aspergillus oryzae, Aspergillus niger, Aspergillus awamori, Aspergillus nidulans, Aspergillus terreus, Cladosphialophora* spp., *Fonsecaea pedrosoi, Fusarium* spp., *Fusarium oxysporum, Fusarium solani, Lichtheimia* spp., *Lichtheimia corymbifera, Lichtheimia ramosa, Myceliophthora* spp., *Myceliophthora thermophila, Rhizopus* spp., *Rhizopus microsporus, Rhizomucor spp., Rhizomucor pusillus, Rhizomucor miehei, Trichoderma* spp., *Trichoderma reesei Trichophyton* spp., *Trichophyton interdigitale,* and *Trichophyton rubrum.* The most preferred filamentous fungal host cell is a strain of the species *Aspergillus niger.*

Several strains of filamentous fungi are readily accessible to the public in a number of culture collections, such as the American Type Culture Collection (ATCC), Chinese Centrum for Industrial Culture Collection (CICC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL), including e.g. the strains *Aspergillus niger* CBS 513.88, CBS124.903, and CICC2462; *Aspergillus oryzae* ATCC 20423, IFO 4177, ATCC 1011, CBS205.89, ATCC 9576, ATCC14488-14491, ATCC 11601 and ATCC12892; *P. chrysogenum* CBS 455.95, *Penicillium citrinum* ATCC 38065, *Penicillium chrysogenum* P2, *Talaromyces emersonii* CBS 124.902, *Acremonium chrysogenum* ATCC 36225 or ATCC 48272, *Trichoderma reesei* ATCC 26921 or ATCC 56765 or ATCC 26921, *Aspergillus sojae* ATCC11906 and *Chrysosporium lucknowense* ATCC44006 and derivatives thereof.

A preferred filamentous fungal host cell is *Aspergillus niger* strain CICC2462, or a strain that is a single colony isolate and/or a derivative of strain CICC2462.

"Yeasts" are herein defined as eukaryotic microorganisms and include all species of the subdivision Eumycotina that predominantly grow in unicellular form. Yeasts may either grow by budding of a unicellular thallus or may grow by fission of the organism. Thus, in one embodiment, a multinucleated cell to be co-transformed is a yeast cell that belongs to a genus selected from the genera including *Candida, Hansenula, Kluyveromyces, Pichia, Saccharomyces, Schizosaccharomyces,* or *Yarrowia,* more preferably a species selected from the species *Kluyveromyces lactis, Saccharomyces cerevisiae, Hansenula polymorpha, Yarrowia lipolytica* and *Pichia pastoris.*

"Yeasts" are herein defined as eukaryotic microorganisms and include all species of the subdivision Eumycotina that predominantly grow in unicellular form. Yeasts may either grow by budding of a unicellular thallus or may grow by fission of the organism. Thus, in one embodiment, a multinucleated cell to be co-transformed is a yeast cell that belongs to a genus selected from the genera including *Candida, Hansenula, Kluyveromyces, Pichia, Saccharomyces, Schizosaccharomyces,* or *Yarrowia,* more preferably a species selected from the species *Kluyveromyces lactis, Saccharomyces cerevisiae, Hansenula polymorpha, Yarrowia lipolytica* and *Pichia pastoris.*

Thus, in one embodiment, the first nucleic acid construct comprises an expression cassette for expression in the cell of a selection marker. A selection marker for use in a filamentous fungal host cell can be selected from the group including, but not limited to, *amdS* (acetamidase), *argB* (ornithine carbamoyltransferase), bar (phosphinothricinacetyltransferase), *ble*A (phleomycin binding), *hyg*B (hygromycinphosphotransferase), *niaD* (nitrate reductase), *pyrG* (orotidine-5'-phosphate decarboxylase), sC (sulfate adenyltransferase), and *trp*C (anthranilate synthase), as well as equivalents from other species. Preferred for use in an *Aspergillus* host cell are the *amdS* (US5876988, US6548285B1) and *pyrG* genes of *A. nidulans* or *A. oryzae* and the *bar* gene of *Streptomyces hygroscopicus.* More preferably an *amdS* gene is used, even more preferably an *amdS* gene from *A. nidulans* or *A. niger.* A most preferred selection marker gene is the A. *nidulans amdS* coding sequence fused to the *A. nidulans gpdA* promoter (see EP 0635574 B1). *AmdS* genes from other filamentous fungi may also be used (US 6548285 B1). A selection marker for use in a yeast host cell may be selected from the group including auxotrophic markers such as *URA3* (orotidine-5'-phosphate decarboxylase), *TRP1* (phosphoribosylanthranilate isomerase), *LEU2* (beta-isopropylmalate dehydrogenase) and *HIS3* (imidazoleglycerol-phosphate dehydratase), antibiotic resistance markers such as *hyg*B (hygromycinphosphotransferase) and *neo* (G418 resistance), as well as *amdS* (acetamidase). Selectable markers can be dominant or recessive or bidirectional. Dominant markers such as antibiotic resistance markers and acetamidases can be used directly in most wild type strains while recessive markers, such as most auxotrophic markers, require that endogenous gene is first inactivated. A bidirectional marker is a marker that not only allows positive selection for the presence of the marker gene but also negative selection, i.e. counter-selection, against the presence of the marker gene by using a compound that is converted to a toxic metabolite by the marker gene product. Examples include fluoroacetamide, that can be used to select against the presence of an acetamidase (*amd*S); 5-fluoroorotic acid (5FOA) that can be used to select against the presence of an orotidine-5'-phosphate decarboxylase (URA3 or *pyr*G); and antiviral drugs, such as acyclovir (ATC: J05AB01) and ganciclovir (ATC: J05AB06) that can be used to select against the presence of a viral thymidine kinase gene, e.g. from the herpes simplex.

In one embodiment, the polynucleotide of interest that is comprised in the second nucleic acid construct encodes at least one polypeptide of interest. The polypeptide can be any polypeptide of interest, e.g. having a biological activity of interest. The term "polypeptide" is not meant herein to refer to a specific length of the encoded product and, therefore, encompasses peptides, oligopeptides, and proteins. Polypeptides further include naturally occurring allelic and engineered variations of the polypeptides, including fusion and hybrid polypeptides. The polypeptide can be native or can be heterologous to the host cell. The polypeptide can be a collagen or gelatin, or a variant or hybrid thereof. The polypeptide can be an antibody or parts thereof, an antigen, a clotting factor, an animal-derived food-protein, an enzyme, a hormone or a hormone variant, a receptor or parts thereof, a regulatory protein, a structural protein, a reporter, or a transport protein, a protein involved in secretion process, a protein involved in folding process, a chaperone, a peptide amino acid transporter, a glycosylation factor, a transcription factor, a synthetic peptide, a synthetic oligopeptide, or an intracellular protein. The intracellular protein can be an enzyme such as, a protease, ceramidases, epoxide hydrolase, aminopeptidase, acylases, aldolase, hydroxylase, aminopeptidase, lipase. The polypeptide can be an enzyme secreted extracellularly. Such enzymes can belong to the groups of oxidoreductase, transferase, hydrolase, lyase, isomerase, ligase, catalase, cellulase, chitinase, cutinase, deoxyribonuclease, dextranase, and esterase. The enzyme can be a carbohydrase, e.g. cellulases such as endoglucanases, β-glucanases, cellobiohydrolases or β-glucosidases, hemicellulases or pectinolytic enzymes such as xylanases, xylosidases, mannanases, galactanases, galactosidases, pectin methyl esterases, pectin lyases, pectate lyases, endo polygalacturonases, exopolygalacturonases rhamnogalacturonases, arabanases, arabinofuranosidases, arabinoxylan hydrolases, galacturonases, lyases, or amylolytic enzymes; hydrolase, isomerase, or ligase, phosphatases such as phytases, esterases such as lipases, proteolytic enzymes, oxidoreductases such as oxidases, transferases, or isomerases. The enzyme can be a phytase. The enzyme can be an aminopeptidase, asparaginase, amylase, carbohydrase, carboxypeptidase, endo-protease, metallo-protease, serine-protease catalase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, protein deaminase, invertase, laccase, lipase, mannosidase, mutanase, oxidase, pectinolytic enzyme, peroxidase, phospholipase, polyphenoloxidase, ribonuclease, transglutaminase, or glucose oxidase, hexose oxidase, monooxygenase. The polypeptide can be animal-derived food-protein of interest such as a hemeprotein, a milk protein or an egg protein, preferably an egg white protein.

In one embodiment, the animal-derived food-protein of interest is a milk protein, preferably a protein present in the milk of cattle (i.e. bovine or *Bos taurus*), buffalo (including water buffalo), goats, sheep or camel, or in the milk of other less common milk animals such as yak, horse, reindeer and donkeys. The protein of interest can be a casein or can be a whey protein such as β-lactoglobulin, α-lactalbumin, bovine serum albumin or an immunoglobulin.

In one embodiment, the animal-derived food-protein of interest is a hemeprotein, preferably a hemeprotein from a non-human animal, more preferably a mammal such as a cow, pig, horse, goat or sheep. Preferred animal-derived hemeproteins include hemoglobin and myoglobin. The animal-derived hemeproteins produced in accordance with the invention can be applied as red heme-bound iron protein in meat substitutes.

In one embodiment, the animal-derived food-protein of interest is an egg protein, i.e. a protein that is present in a bird's egg. The term "bird" as used herein includes both domesticated birds and non-domesticated birds such as wild life birds. Birds e.g. include poultry, fowl, waterfowl, game bird, ratite (e.g., flightless bird), chicken (*Gallus gallus domesticus*), quail, turkey, duck, ostrich (*Struthio camelus*), Somali ostrich (*Struthio molybdophanes*), goose, gull, guineafowl, pheasant, emu (*Dromaius novaehollandiae*), American rhea (*Rhea americana*), Darwin's rhea (*Rhea pennata*) and kiwi. Preferably, the animal-derived food-protein of interest is an egg white protein. The egg white protein can be an egg white protein selected from the group consisting of ovalbumin, ovotransferrin, ovomucoid, G162M F167A ovomucoid, ovoglobulin G2, ovoglobulin G3, α-ovomucin, β-ovomucin, lysozyme, ovoinhibitor, ovoglycoprotein, flavoprotein, ovomacroglobulin, ovostatin, cystatin, avidin, ovalbumin related protein X and ovalbumin related protein Y (see e.g. US2018/0355020). A particularly preferred egg white protein is ovalbumin.

In one embodiment, the ovalbumin that is encoded in the expression cassette of the invention comprises an amino acid sequence with at least 50, 55, 60, 65, 70, 75, 80, 85, 90, 95, 96, 97, 98, 99 or 100% identity to the amino acid sequence of an ovalbumin from a bird selected from the group consisting of chicken, pelican, quail, pigeon, ostrich, plover, turkey, duck, goose, gull, guineafowl, junglefowl, peafowl, partridge, pheasant, emu, rhea and kiwi, of which chicken, pigeon, pelican and quail are preferred.

In one embodiment, a polypeptide of interest can also be a fused or hybrid polypeptide to which another polypeptide is fused at the N-terminus or the C- terminus of the polypeptide or fragment thereof. A fused polypeptide is produced by fusing a nucleic acid sequence (or a portion thereof) encoding one polypeptide to a nucleic acid sequence (or a portion thereof) encoding another polypeptide.

### Examples

### Methods and materials

### Expression cassette of gene of interest

The GOI expression cassette consists of the *Aspergillus niger* endogenous *glaA* promoter, the GOI (hen ovalbumin) ORF, the endogenous *glaA* terminator and is flanked by an integration flank that is homologous to the 3' downstream sequence of *glaA* for targeted integration of the expression construct (3' *glaA*-GOI).

### Selection marker

The selection marker cassette consists of a constitutively active promoter (*gpdA*), the *amdS* (acetamidase) open reading frame for selection on acetamide and a *trpC* terminator (sequences based on *Aspergillus nidulans* FGSC 4). The *PgpdA-amdS-TtrpC* cassette can be flanked upstream and downstream with approximately 500 bp repeatable, endogenous DNA sequence to optionally loop out the selection marker in a transformant by growing it on fluoroacetamide. The repeatable element can be derived for instance from the 3' downstream region of the *Aspergillus niger* Endopolygalacturonase B gene; *pgaB* (GenBank accession number: Y18805.1). Furthermore, the selection cassette can contain a single or a double cross-over integration flank, for instance a single integration flank consisting of a 1500 bp sequence endogenous to the 5' downstream of the *pgaB* sequence of A. *niger* (see Figure 1).

### General molecular biology techniques

Unless indicated otherwise, the methods used are standard techniques. Examples of suitable general methodology textbooks include Sambrook et al., Molecular Cloning, a Laboratory Manual (1989) and Ausubel et al., Current Protocols in Molecular Biology (1995), John Wiley & Sons, Inc.

### Strains

*Aspergillus niger* strain BZASNI.22a is a single colony isolate of CICC2462. BZASNI.22a was characterized by rDNA ITS sequencing and by resequencing of the genome (BaseClear B.V., the Netherlands). A. *niger* BZASNI.48 is a single colony isolate of BZASNI.22a, which is able to sporulate on inducible medium.

### Plasmids and oligonucleotide primers

Plasmids used in the Examples are listed in Table 1. Primers used in the Examples are listed in Table 2.

**Table 1 Primer list**

| **primer #** | **Description** | **Purpose** | **SEQ ID NO** |
|---|---|---|---|
| 612 | P*gpdA_*fw | Construction - Gibson assembly | 1 |
| 460 | *pgaB-*P6_rv | Construction - Gibson assembly | 2 |
| 456 | *pgaB-*P3_fw | Construction - Gibson assembly | 3 |
| 458 | *pgaB-*P4_rv | Construction - Gibson assembly | 4 |
| 462 | *pgaB-*P8_rv | Construction - Gibson assembly | 5 |
| 453 | *pgaB-*P1_fw | Construction - Gibson assembly | 6 |
| 455 | *pgaB-P2_rv* | Construction - Gibson assembly | 7 |
| 431 | pUC19_fw | Construction - Gibson assembly | 8 |
| 432 | pUC19_rv | Construction - Gibson assembly | 9 |
| 738 | *pgaB-*amdS_fw | Transformation - amdS | 10 |
| 739 | *pgaB-*amdS_rv | Transformation - amdS | 11 |
| 787 | glaA-GOI_fw | Transformation GOI | 12 |
| 789 | *glaA-*GOI_rv | Transformation GOI | 13 |
| 400 | *amdS_*fw | Genotyping colony PCR | 14 |
| 401 | *amdS_*rv | Genotyping colony PCR | 15 |
| 392 | GOI_fw | Genotyping colony PCR | 16 |
| 732 | GOI_rv | Genotyping colony PCR | 17 |

**Table 2 Plasmid list**

| **Plasmid** | **Characteristics** | **SEQ ID NO** |
|---|---|---|
| pBZ0026 | Vector with PgpdA-amdSORF-TtrpC (*A. nidulans FGSC 4*) | 18 |
| pUC19 | Ampicillin resistance marker, E.coli plasmid | 19 |
| pBZ0070 | pUC19 with 5'pgaB-PgpdA-amdS ORF-TtrpC | 20 |
| pBZ0061 | Vector with 3'glaA-GOI | 21 |

### Kits

For the purification of PCR fragments and extraction of DNA fragments from agarose gel the Wizard^{®} SV Gel and PCR clean-up system (Promega) was used. Purified PCR products were transformed in the pCR^{™}Blunt II-TOPO^{®} vector from the Zero BlunF^{™} TOPO^{™} PCR Cloning Kit (Thermo Fisher Scientific). Amplified plasmids were isolated with the Qiaprep spin miniprep kit.

### Enzymes

Enzymes for DNA manipulations (e.g. digestions, Golden Gate Reactions, Gibson assembly) were obtainable from New England Biolabs and were used according to the manufacturer's protocols.

### PCR amplification

All DNA polymerases are from New England Biolabs Inc. and PCR is performed according to manufacturer's protocol. DNA fragments for plasmid construction are PCR amplified using Phusion^{®} High-Fidelity DNA Polymerase. Linear DNA is generated for transformation using Q5^{®} High-Fidelity DNA Polymerase. The presence of the GOI and the *amdS* gene during purification of segregants is analyzed using OneTaq^{®} DNA Polymerase.

### Colony PCR

Protoplasts are generated from single colony mycelium using 0.375 mL Zymolyase R (Zymoresearch) in 25 µL 0.1 M sodium phosphate buffer pH7.5. After 1 hour incubation the protoplasts are disrupted by heating for 5 minutes at 100°C. 75 µL of DNA dilution buffer (10 mM NaCl, 100 mM Tris/HCI pH 7.5 and 10 mM EDTA) is added to the protoplasts to neutralize cell-derived material and 100 µL HPLC water is added to dilute the DNA and optional polymerase-interfering substances. 2.5 µL of template is used for PCR amplification.

### Media

Media used in the construction of expression cassettes: liquid LB (10 g/l Tryptone, 5 g/l Yeast extract, 5 g/l NaCl) and solid LB (addition of 15 g/l agar) with the appropriate antibiotic (Neomycin 50 µg/ml or ampicillin 100 µg/ml).

Media used for transformation and selection of the *A. niger* transformants: Bottom agar (0.54 g/L NH₄Cl, 44.73 g/L KCl, 1 mL/L Vishniac's trace elements solution (per litre; 10 g EDTA; 4.4 g ZnSO₄.7H₂O; 1.0 g MnCl₂·4H₂O; 0.32 g CoCl₂·6H₂O; 0.32 g CuSO₄·5H₂O; 0.22 g 15 (NH₄)₆Mo₇O₂₄·4H₂O; 1.47 g CaCl₂·2H₂O; 1.0 g FeSO₄·7H₂O), 11 g/L D-glucose, 6 g/L agar and initial pH of 6) with the appropriate selection (10mM acetamide in combination with 15 mM caesium chloride), Top agar (0.54 g/L NH₄Cl, 1 mL/L Vishniac's trace elements solution, 11 g/L D-glucose, 6 g/L agar and initial pH of 6) with the appropriate selection (10 mM acetamide in a combination with 15 mM caesium chloride) and minimal medium (0.54 g/L NH₄Cl, 1 mL/L Vishniac's trace elements solution, 11 g/L D-glucose, 14 g/L agar and initial pH of 6) with the appropriate selection (10 mM acetamide in a combination with 15mM caesium chloride). Sporulation agar (0.54 g/L NH₄Cl, 1 mL/L Vishniac's trace elements solution, 11 g/L D-glucose, 14 g/L agar and 44.73 g/L KCL and initial pH of 6) was used to purify the *A. niger* transformants. Potato Dextrose agar (PDA) of Sigma was used according to manufacturer's protocol.

### Transformation of A. niger

The transformation of *A. niger* was done according to the protocol based on Balance *et al.* (Ballance et al., 1983, Biochem. Biophys. Res. Comm. 112, 284-289). Transformation agar plate composition was based on Arentshorst *et al.* (Arentshorst et al., 2012, Methods Mol. Biol. 835:133-50.).

### Isolation of homokaryotic segregants

In the segregant purification approach there are two different ways to transfer the fungus from one plate to another, spore transfer and mycelium transfer. Spores are harvested with physiologic salt with Tween 80 (0.9% NaCl, 0.004% Tween80) and filtered through two-layer miracloth filters (Merck). Spores are counted with the spore Bürker counter (Marienfeld) and diluted to result in approximately 100 spores/plate. Mycelium from a single colony is crushed in 100 µL physiological salt with Tween 80 using a toothpick and streaked on another plate.

### Example 1 - construction of expression and selection marker gene cassettes

The *amdS* selection cassette is constructed with PCR amplification, Golden gate cloning and Gibson assembly (Figure 1A). The *PgpdA-amdS-TtrpC* cassette is PCR amplified using primers 612 + 460 with pBZ0026 as a template generating fragment c. The repeatable elements b and d and the 5' *pgaB* integration flank a, are amplified using primers 456 + 458, 456 + 462 and 453 + 455, respectively with BZASNI.22a genomic DNA as template. The pUC19 backbone is PCR amplified using primers 431 + 432. All PCR amplified fragments contain 25 bp overlapping flanks with 100% sequence identity to adjacent fragments to allow Gibson assembly into the final plasmid pBZ0070 (Figure 1.A). The final construct was verified by restriction enzyme analysis and DNA sequencing. The entire marker expression cassette with the integration site and repeatable DNA elements used for transformation was PCR amplified using primers 738 + 739.

The 3' *glaA* targeted GOI expression cassette (with the hen ovalbumin cDNA as the gene of interest) was constructed using PCR amplification, Golden gate cloning and Gibson assembly (Figure 1B). The transformation construct was PCR amplified using primers 787 + 789.

### Example 2 - co-transformation of GOI expression cassette with a single cross-over marker selection cassette and selection of segregants

The goal of the segregant purification process is to identify and select transformed nuclei that obtained the GOI but which did not obtain the *amdS* selection cassette. This has been done using various phenotypic and genotypic screening methods employing different kinds of media (Figure 2A). In each step, there is a specific selection for a desired phenotype or genotype. Only a selection of transformants was used to continue with to the next step (Figure 2B).

The results of individual selection steps were as follows:
a. Step 1: Transformants growing on acetamide medium were screened for the presence of the GOI using colony PCR (primer 392 + 732). 16 out of 23 colonies showed to contain the GOI and mycelium was plated on sporulation inducible agar (Figure 3).
b. Step 2: Nuclei were forced to segregate into single spores by plating fragmented mycelium onto induced sporulation agar. 16 out of 16 selected transformants showed to be sporulating and 10 - 100 spores were transferred to acetamide medium.
c. Step 3: Phenotypic selection for the absence of the *amdS* gene by selecting small colonies from big ones (Figure 4). 8 out of 16 transformants consisted of at least one small colony. For each transformant with small colonies, 3 colonies (named colony A-C) were selected to continue with. If possible, one additional bigger colony was selected as well (named colony D).
d. Step 4 Small colonies were transferred to Potato Dextrose Agar (PDA) to recover enough biomass for genotyping. The colonies were screened for the presence of the GOI (primer 392 + 732) and the *amdS* gene (primer 400 + 401). In 4 out of 8 transformants there was at least one colony being *amdS-* and ova+ (Figure 5). For 56.04 (A, B and C), 56.07 (only segregant B), 56.08 (A, B and C) and 56.23 (A and B). All segregants (A-D) of these four selected transformants were plated on sporulation inducible agar by mycelium transfer.
e. Step 5: 10 - 100 spores are plated on acetamide medium for phenotypic analysis and on PDA to perform colony PCR for genotypic analysis. For each segregant; ≥ 6 colonies were analyzed (Figure 6). Almost all segregants, being *amdS-* and ova+ in step 4, were verified in all 8 spore derivatives of these strains. Only segregants originating from a bigger colony in step 3 (colony D) show variation in phenotype and genotype.

Using the segregation approach we have obtained 10 segregants originating from 4 original colonies on transformation plate having the desired *amdS-* GOI+ genotype. This generates a segregation efficiency of 4 out of 16 (e.g. ± 25% of co-transformants). Using similar combinations of *amdS* constructs and GOI constructs we obtained segregation efficiencies of 20-40% (data not shown).

### References:

Arentshorst M.et al (2012) Using non-homologous end-joining-deficient strains for functional gene analyses in filamentous fungi. Methods Mol Biol. 835:133-50.
Roper, M. et al. (2011). Nuclear and genome dynamics in multinucleate Ascomycete fungi. Curr Biol. 21 (18): R786-R793.
Strom, N.B. et al. (2016). Two genomes are better than one: history, genetics, and biotechnological applications of fungal heterokaryons. Fungal Biol Biotechnol 3:4.
Mayer, V. et al. (2007). Highly efficient gene targeting in the Aspergillus niger kusA mutant. J. Biotech. 128: 770-775.
Ruiz-Diez, B. (2002). A review: Strategies for the transformation of filamentous fungi. J Appl Microbiol 92: 189-195.
Meyer, V. et al. (2011). Aspergillus as a multi-purpose cell factory: current status and perspectives. Biotechnol Lett 33: 469-476.
EFSA Scientific Opinion (2011). Guidance on the risk assessment of genetically modified microorganisms and their products intended for food and feed use. EFSA Journal 9 (6): 2193.
EFSA Scientific Opinion (2018). Guidance of the characterisation of microorganisms used as feed additives or as a production organisms. EFSA Journal 16 (3): 5206.
Buxton, P.F. et al. (1989). US4885249 patent - *Aspergillus niger* transformation system.
Swinkels, B.W et al. (2003). US6548285 - Polynucleotides encoding *Aspergillus niger* and *Penicillium chrysogenum* acetamidases and methods of use as selectable markers.
Selten, G.C.M. et al. (1999). US5876988 - Selection marker gene free recombinant strains: method for obtaining them and the use of these strains.

## Claims

1. A method for obtaining a selection marker gene-free recombinant strain comprising the following steps:
a) co-transformation of a multinucleated cell with at least two separate nucleic acid constructs, wherein the first nucleic acid construct comprises an expression cassette for expression in the cell of a selection marker, and wherein a second nucleic acid construct comprises a gene of interest;
b) selection of the multinucleated cell as obtained in a) for a transformed heterokaryotic cell expressing the selection marker;
c) screening of the transformed heterokaryotic cell as obtained in b) for a heterokaryotic cell of which at least one nucleus comprises the gene of interest;
d) segregating the heterokaryotic cell of which at least one nucleus comprises the gene of interest as obtained in c) into one or more homokaryotic cells; and,
e) screening and/or selecting the one or more homokaryotic cells as obtained in d) for at least one cell that comprises the gene of interest and that lacks the selection marker, to obtain the selection marker gene-free recombinant strain.

2. A method according to claim 1, wherein in step d) the heterokaryotic cell is segregated into one or more homokaryotic cells by sporulating the heterokaryotic cell.

3. A method according to claim 1 or 2, wherein in step e) the one or more homokaryotic cells as obtained in d) are subjected to a differential selection regime that allows to distinguish on the basis of a phenotypic difference between a cell that lacks the selection marker and a cell that expresses the selection marker.

4. A method according to claim 3, wherein the phenotypic difference is a difference in growth properties expressed under the differential selection regime.

5. A method according to claim 4, wherein the differential selection regime is on a solid medium and a cell that lacks the selection marker is distinguished from a cell that expresses the selection marker on the basis of colony size or shape.

6. A method according to claim 1 or 2, wherein the selection marker is a bidirectional marker and in step e) the one or more homokaryotic cells as obtained in d) are subjected to a counter-selection regime that selects against cells expressing the selection marker.

7. A method according to any one of the preceding claims, wherein the at least one cell that comprises the gene of interest and that lacks the selection marker, as obtained in step e), is subjected to a further round of segregation into one or more homokaryotic cells, preferably by sporulation, and optionally followed by at least one of screening and selection to identify a selection marker gene-free recombinant strain having the desired genotype.

8. A method according to any one of the preceding claims, wherein at least one of the first and second nucleic acid constructs are integrative nucleic acid constructs.

9. A method according to any one of the preceding claims, wherein the multinucleated cell to be co-transformed with the at least at least two nucleic acid constructs is a cell of a heterokaryotic organism.

10. A method according to any one of the preceding claims, wherein the multinucleated cell to be co-transformed with the at least at least two nucleic acid constructs is a cell of a filamentous fungus or a yeast

11. A method according to claim 10, wherein the filamentous fungus belongs to a genus selected from the genera including *Alternaria, Apophysomyces, Aspergillus, Cladosphialophora, Fonsecaea, Fusarium, Lichtheimia, Myceliophthora, Rhizopus, Rhizomucor, Trichoderma* and *Trichophyton,* or wherein the yeast belongs to a genus selected from the genera including *Candida, Hansenula, Kluyveromyces, Pichia, Saccharomyces, Schizosaccharomyces,* and *Yarrowia.*

12. A method according to claim 10 or 11, wherein the filamentous fungus belongs a species selected from *Alternaria alternata, Apophysomyces variabilis, Aspergillus* spp., *Aspergillus fumigatus, Aspergillus flavus, Aspergillus oryzae, Aspergillus niger, Aspergillus awamori, Aspergillus nidulans, Aspergillus terreus, Cladosphialophora* spp., *Fonsecaea pedrosoi, Fusarium* spp., *Fusarium oxysporum, Fusarium solani, Lichtheimia* spp., *Lichtheimia corymbifera, Lichtheimia ramosa, Myceliophthora* spp., *Myceliophthora thermophila, Rhizopus* spp., *Rhizopus microsporus, Rhizomucor spp., Rhizomucor pusillus, Rhizomucor miehei, Trichoderma* spp., *Trichoderma reesei, Trichophyton* spp., *Trichophyton interdigitale,* and *Trichophyton rubrum,* of which the species *Aspergillus niger* is most preferred, or wherein the yeast belongs to a species selected from the species *Kluyveromyces lactis, Saccharomyces cerevisiae, Hansenula polymorpha, Yarrowia lipolytica* and *Pichia pastoris.*

13. A method according to any one of the preceding claims, wherein the selection marker encodes an acetamidase, wherein preferably the selection marker is an *Aspergillus amdS* gene.

14. A method according to any one of the preceding claims, wherein the gene of interest encodes a polypeptide of interest or a DNA or RNA molecule of interest.

15. A method according to claim 14, wherein the polypeptide of interest is selected from the group consisting of: an antibody or antigen-binding fragment thereof, an antigen, a clotting factor, an animal-derived food-protein, an enzyme, a hormone or a hormone variant, a receptor or parts thereof, a regulatory protein, a structural protein, a reporter, or a transport protein, a protein involved in secretion process, a protein involved in folding process, a chaperone, a peptide amino acid transporter, a glycosylation factor, a transcription factor, a synthetic peptide or oligopeptide and an intracellular protein.


SEQUENCE LISTING

<110>  The Protein Brewery B.V.

<120>  Methods for generating selection marker-free transformants of
       heterokaryotic organisms

<130>  P6099410EP

<160>  21

<170>  PatentIn version 3.5

<210>  1
<211>  19
<212>  DNA
<213>  Artificial Sequence

<220>
<223>  Primer

<400>  1
gagctctgta cagtgaccg                                                    19


<210>  2
<211>  50
<212>  DNA
<213>  Artificial Sequence

<220>
<223>  Primer

<400>  2
tcaacacagc tggccagaga cccgtaccta cgatgaatgt gtgtcctgta                  50


<210>  3
<211>  20
<212>  DNA
<213>  Artificial Sequence

<220>
<223>  Primer

<400>  3
acgggtctct ggccagctgt                                                   20


<210>  4
<211>  50
<212>  DNA
<213>  Artificial Sequence

<220>
<223>  Primer

<400>  4
agtcaccggt cactgtacag agctctttga gtttggcggc ggaagattag                  50


<210>  5
<211>  50

<212>  DNA
<213>  Artificial Sequence

<220>
<223>  Primer

<400>  5
attctgtgga taaccgtatt accgcaactt ctccgtgatg ctcgtgttgc                50


<210>  6
<211>  50
<212>  DNA
<213>  Artificial Sequence

<220>
<223>  Primer

<400>  6
aataggcgta tcacgaggcc ctttcaatgc tggaaggagc ttgcctttcg                50


<210>  7
<211>  50
<212>  DNA
<213>  Artificial Sequence

<220>
<223>  Primer

<400>  7
tcaacacagc tggccagaga cccgtgatga taatgaagga atggaacctg                50


<210>  8
<211>  20
<212>  DNA
<213>  Artificial Sequence

<220>
<223>  Primer

<400>  8
gaaagggcct cgtgatacgc                                                 20


<210>  9
<211>  20
<212>  DNA
<213>  Artificial Sequence

<220>
<223>  Primer

<400>  9
gcggtaatac ggttatccac                                                 20


<210>  10
<211>  25
<212>  DNA
<213>  Artificial Sequence

<220>
<223>  Primer

<400>  10
aatgctggaa ggagcttgcc tttcg                                          25


<210>  11
<211>  20
<212>  DNA
<213>  Artificial Sequence

<220>
<223>  Primer

<400>  11
agacgcaggt ggaggctttg                                                20


<210>  12
<211>  24
<212>  DNA
<213>  Artificial Sequence

<220>
<223>  Primer

<400>  12
gatcattgat ccaccgccca cgag                                           24


<210>  13
<211>  24
<212>  DNA
<213>  Artificial Sequence

<220>
<223>  Primer

<400>  13
atttagtggc agatggcatc acag                                           24


<210>  14
<211>  20
<212>  DNA
<213>  Artificial Sequence

<220>
<223>  Primer

<400>  14
gcctcaatcc tgggaagaac                                                20


<210>  15
<211>  20
<212>  DNA
<213>  Artificial Sequence

<220>
<223>  Primer

<400>  15
gcccattgat gtttcgtagc                                                   20


<210>  16
<211>  20
<212>  DNA
<213>  Artificial Sequence

<220>
<223>  Primer

<400>  16
tgactcagcc gacgagatcc                                                   20


<210>  17
<211>  20
<212>  DNA
<213>  Artificial Sequence

<220>
<223>  Primer

<400>  17
tcattcgcaa cgtgctccag                                                   20


<210>  18
<211>  5073
<212>  DNA
<213>  Artificial Sequence

<220>
<223>  Plasmid pBZ0026

<400>  18
ggaggagctc tgtacagtga ccggtgactc tttctggcat gcggagagac ggacggtcgc      60

agagaggagg gctgagtaat aagcgcactc atgtcagctc tggcgctctg aggtgcagtg     120

gatgattatt aatccgggac cggccgcccc tccgccccga agtggaaagg ctggtgtgcc     180

cctcgttgac caagaatcta ttgcatcatc ggagaatatg gagcttcatc gaatcaccgg     240

cagtaagcga aggagaatgt gaagccaggg gtgtatagcc gtcggcgaaa tagcatgcca     300

ttaacctagg tacagaagtc caattgcttc cgatctggta aaagattcac gagatagtac     360

cttctccgaa gtaggtagag cgagtacccg gcgcgtaagc tccctaattg gcccatccgg     420

catctgtagg gcgtccaaat atcgtgcctc tcctgctttg cccggtgtat gaaaccggaa     480

aggccgctca ggagctggcc agcggcgcag accgggaaca caagctggca gtcgacccat     540

ccggtgctct gcactcgacc tgctgaggtc cctcagtccc tggtaggcag ctttgccccg     600

tctgtccgcc cggtgtgtcg gcggggttga caaggtcgtt gcgtcagtcc aacatttgtt     660

gccatatttt cctgctctcc ccaccagctg ctcttttctt ttctctttct tttcccatct     720

tcagtatatt catcttccca tccaagaacc tttatttccc ctaagtaagt actttgctac     780

atccatactc catccttccc atcccttatt cctttgaacc tttcagttcg agctttccca 840

cttcatcgca gcttgactaa cagctacccc gcttgagcag acatcacaaa tgcctcaatc 900

ctgggaagaa ctggccgctg ataagcgcgc ccgcctcgca aaaaccatcc ctgatgaatg 960

gaaagtccag acgctgcctg cggaagacag cgttattgat ttcccaaaga aatcggggat 1020

cctttcagag gccgaactga agatcacaga ggcctccgct gcagatcttg tgtccaagct 1080

ggcggccgga gagttgacct cggtggaagt tacgctagca ttctgtaaac gggcagcaat 1140

cgcccagcag ttaacaaact gcgcccacga gttcttccct gacgccgctc tcgcgcaggc 1200

aagggaactc gatgaatact acgcaaagca caagcgcccc gttggtccac tccatggcct 1260

ccccatctct ctcaaagacc agcttcgagt caagggctac gaaacatcaa tgggctacat 1320

ctcatggcta aacaagtacg acgaagggga ctcggttctg acaaccatgc tccgcaaagc 1380

cggtgccgtc ttctacgtca agacctctgt cccgcagacc ctgatggtct gcgagacagt 1440

caacaacatc atcgggcgca ccgtcaaccc acgcaacaag aactggtcgt gcggcggcag 1500

ttctggtggt gagggtgcga tcgttgggat tcgtggtggc gtcatcggtg taggaacgga 1560

tatcggtggc tcgattcgag tgccggccgc gttcaacttc ctgtacggtc taaggccgag 1620

tcatgggcgg ctgccgtatg caaagatggc gaacagcatg gagggtcagg agacggtgca 1680

cagcgttgtc gggccgatta cgcactctgt tgaggacctc cgcctcttca ccaaatccgt 1740

cctcggtcag gagccatgga aatacgactc caaggtcatc cccatgccct ggcgccagtc 1800

cgagtcggac attattgcct ccaagatcaa gaacggcggg ctcaatatcg gctactacaa 1860

cttcgacggc aatgtccttc cacaccctcc tatcctgcgc ggcgtggaaa ccaccgtcgc 1920

cgcactcgcc aaagccggtc acaccgtgac cccgtggacg ccatacaagc acgatttcgg 1980

ccacgatctc atctcccata tctacgcggc tgacggcagc gccgacgtaa tgcgcgatat 2040

cagtgcatcc ggcgagccgg cgattccaaa tatcaaagac ctactgaacc cgaacatcaa 2100

agctgttaac atgaacgagc tctgggacac gcatctccag aagtggaatt accagatgga 2160

gtaccttgag aaatggcggg aggctgaaga aaaggccggg aaggaactgg acgccatcat 2220

cgcgccgatt acgcctaccg ctgcggtacg gcatgaccag ttccggtact atgggtatgc 2280

ctctgtgatc aacctgctgg atttcacgag cgtggttgtt ccggttacct ttgcggataa 2340

gaacatcgat aagaagaatg agagtttcaa ggcggttagt gagcttgatg ccctcgtgca 2400

ggaagagtat gatccggagg cgtaccatgg ggcaccggtt gcagtgcagg ttatcggacg 2460

gagactcagt gaagagagga cgttggcgat tgcagaggaa gtggggaagt tgctgggaaa 2520

tgtggtgact ccataggctt tgatttaata gctccatgtc aacaagaata aaacgcgttt 2580

cgggtttacc tcttccagat acagctcatc tgcaatgcat taatgcattg gacctcgcaa 2640

ccctagtacg cccttcaggc tccggcgaag cagaagaata gcttagcaga gtctattttc 2700

attttcggga gacgagatca agcagatcaa cggtcgtcaa gagacttacg agactgagga 2760

atccgctctt ggctccacgc gactatatat ttgtctctaa ttgtactttg acatgctcct 2820

cttctttact ctgatagctt gactatgaaa attccgtcac cagcccctgg gttcgcaaag 2880

ataattgcac tgtttcttcc ttgaactctc aagcctacag gacacacatt catcgtaggt 2940

ccattcctgt agtcttctta attaagacgt cagaattctc gaggcggccg catgtgcgtc 3000

tccctatagt gagtcgtatt aatttcgcgg gcggaacccc tatttgttta tttttctaaa 3060

tacattcaaa tatgtatccg ctcatgagta gcaccaggcg tttaagggca ccaataactg 3120

ccttaaaaaa attacgcccc gccctgccac tcatcgcagt actgttgtaa ttcattaagc 3180

attctgccga catggaagcc atcacaaacg gcatgatgaa cctgaatcgc cagcggcatc 3240

agcaccttgt cgccttgcgt ataatatttg cccatggtga aaacgggggc gaagaagttg 3300

tccatattgg ccacgtttaa atcaaaactg gtgaaactca cccagggatt ggctgagacg 3360

aaaaacatat tctcaataaa ccctttaggg aaataggcca ggttttcacc gtaacacgcc 3420

acatcttgcg aatatatgtg tagaaactgc cggaaatcgt cgtggtattc actccagagc 3480

gatgaaaacg tttcagtttg ctcatggaaa acggtgtaac aagggtgaac actatcccat 3540

atcaccagct caccgtcttt cattgccata cggaattccg gatgagcatt catcaggcgg 3600

gcaagaatgt gaataaaggc cggataaaac ttgtgcttat ttttctttac ggtctttaaa 3660

aaggccgtaa tatccagctg aacggtctgg ttataggtac attgagcaac tgactgaaat 3720

gcctcaaaat gttctttacg atgccattgg gatatatcaa cggtggtata tccagtgatt 3780

tttttctcca ttttagcttc cttagctcct gaaaatctcg ataactcaaa aaatacgccc 3840

ggtagtgatc ttatttcatt atggtgaaag ttggaacctc ttacgtgccg atcaacgtct 3900

cattttcgcc aaaagttgtc atgaccaaaa tcccttaacg tgagttttcg ttccactgag 3960

cgtcagaccc cgtagaaaag atcaaaggat cttcttgaga tccttttttt ctgcgcgtaa 4020

tctgctgctt gcaaacaaaa aaaccaccgc taccagcggt ggtttgtttg ccggatcaag 4080

agctaccaac tctttttccg aaggtaactg gcttcagcag agcgcagata ccaaatactg 4140

ttcttctagt gtagccgtag ttaggccacc acttcaagaa ctctgtagca ccgcctacat 4200

acctcgctct gctaatcctg ttaccagtgg ctgctgccag tggcgataag tcgtgtctta 4260

ccgggttgga ctcaagacga tagttaccgg ataaggcgca gcggtcgggc tgaacggggg 4320

gttcgtgcac acagcccagc ttggagcgaa cgacctacac cgaactgaga tacctacagc 4380

gtgagctatg agaaagcgcc acgcttcccg aagggagaaa ggcggacagg tatccggtaa 4440

gcggcagggt cggaacagga gagcgcacga gggagcttcc agggggaaac gcctggtatc 4500

tttatagtcc tgtcgggttt cgccacctct gacttgagcg tcgatttttg tgatgctcgt 4560

caggggggcg gagcctatgg aaaaacgcca gcaatgcggc ctttttacgg ttcctggcct 4620

tttgctggcc ttttgctcac atgttctttc ctgcgttatc ccctgattct gtggataacc 4680

gtattaccgc ctttgagtga gctgataccg ctcgccgcag ccgaacgacc gagcgcagcg 4740

agtcagtgag cgaggaagcc gaaaaatcaa taatcagaca acaagatgtg cgaactcgat 4800

attttacacg actctcttta ccaattctgc cccgaattac acttaaaacg actcaacagc 4860

ttaacgttgg cttgccacgc attacttgac tgtaaaactc tcactcttac cgaacttggc 4920

cgtaacctgc caaccaaagc gagaacaaaa cataacatca aacgaatcga ccgattgtta 4980

ggtaatcgtc acctgcagga aggtttaaac gcatttaggt gacactatag aagtgtgtat 5040

cgctcgaggg atccgaattc gaagacttgg tac 5073

<210> 19
<211> 1924
<212> DNA
<213> Artificial Sequence

<220>
<223> Plasmid pUC19

<400> 19
gcggtaatac ggttatccac agaatcaggg gataacgcag gaaagaacat gtgagcaaaa 60

ggccagcaaa aggccaggaa ccgtaaaaag gccgcgttgc tggcgttttt ccataggctc 120

cgcccccctg acgagcatca caaaaatcga cgctcaagtc agaggtggcg aaacccgaca 180

ggactataaa gataccaggc gtttccccct ggaagctccc tcgtgcgctc tcctgttccg 240

accctgccgc ttaccggata cctgtccgcc tttctccctt cgggaagcgt ggcgctttct 300

catagctcac gctgtaggta tctcagttcg gtgtaggtcg ttcgctccaa gctgggctgt 360

gtgcacgaac cccccgttca gcccgaccgc tgcgccttat ccggtaacta tcgtcttgag 420

tccaacccgg taagacacga cttatcgcca ctggcagcag ccactggtaa caggattagc 480

agagcgaggt atgtaggcgg tgctacagag ttcttgaagt ggtggcctaa ctacggctac 540

actagaagaa cagtatttgg tatctgcgct ctgctgaagc cagttacctt cggaaaaaga 600

gttggtagct cttgatccgg caaacaaacc accgctggta gcggtggttt ttttgtttgc 660

aagcagcaga ttacgcgcag aaaaaaagga tctcaagaag atcctttgat cttttctacg 720

gggtctgacg ctcagtggaa cgaaaactca cgttaaggga ttttggtcat gagattatca 780

aaaaggatct tcacctagat ccttttaaat taaaaatgaa gttttaaatc aatctaaagt 840

atatatgagt aaacttggtc tgacagttac caatgcttaa tcagtgaggc acctatctca 900

gcgatctgtc tatttcgttc atccatagtt gcctgactcc ccgtcgtgta gataactacg 960

atacgggagg gcttaccatc tggccccagt gctgcaatga taccgcgaga cccacgctca 1020

ccggctccag atttatcagc aataaaccag ccagccggaa gggccgagcg cagaagtggt 1080

cctgcaactt tatccgcctc catccagtct attaattgtt gccgggaagc tagagtaagt 1140

agttcgccag ttaatagttt gcgcaacgtt gttgccattg ctacaggcat cgtggtgtca 1200

cgctcgtcgt ttggtatggc ttcattcagc tccggttccc aacgatcaag gcgagttaca 1260

tgatccccca tgttgtgcaa aaaagcggtt agctccttcg gtcctccgat cgttgtcaga 1320

agtaagttgg ccgcagtgtt atcactcatg gttatggcag cactgcataa ttctcttact 1380

gtcatgccat ccgtaagatg cttttctgtg actggtgagt actcaaccaa gtcattctga 1440

gaatagtgta tgcggcgacc gagttgctct tgcccggcgt caatacggga taataccgcg 1500

ccacatagca gaactttaaa agtgctcatc attggaaaac gttcttcggg gcgaaaactc 1560

tcaaggatct taccgctgtt gagatccagt tcgatgtaac ccactcgtgc acccaactga 1620

tcttcagcat cttttacttt caccagcgtt tctgggtgag caaaaacagg aaggcaaaat 1680

gccgcaaaaa agggaataag ggcgacacgg aaatgttgaa tactcatact cttccttttt 1740

caatattatt gaagcattta tcagggttat tgtctcatga gcggatacat atttgaatgt 1800

atttagaaaa ataaacaaat aggggttccg cgcacatttc cccgaaaagt gccacctgac 1860

gtctaagaaa ccattattat catgacatta acctataaaa ataggcgtat cacgaggccc 1920

tttc 1924

<210> 20
<211> 8361
<212> DNA
<213> Artificial Sequence

<220>
<223> Plasmid pBZ0070

<400> 20
gcggtaatac ggttatccac agaatcaggg gataacgcag gaaagaacat gtgagcaaaa 60

ggccagcaaa aggccaggaa ccgtaaaaag gccgcgttgc tggcgttttt ccataggctc 120

cgcccccctg acgagcatca caaaaatcga cgctcaagtc agaggtggcg aaacccgaca 180

ggactataaa gataccaggc gtttccccct ggaagctccc tcgtgcgctc tcctgttccg 240

accctgccgc ttaccggata cctgtccgcc tttctccctt cgggaagcgt ggcgctttct 300

catagctcac gctgtaggta tctcagttcg gtgtaggtcg ttcgctccaa gctgggctgt 360

gtgcacgaac cccccgttca gcccgaccgc tgcgccttat ccggtaacta tcgtcttgag 420

tccaacccgg taagacacga cttatcgcca ctggcagcag ccactggtaa caggattagc 480

agagcgaggt atgtaggcgg tgctacagag ttcttgaagt ggtggcctaa ctacggctac 540

actagaagaa cagtatttgg tatctgcgct ctgctgaagc cagttacctt cggaaaaaga 600

gttggtagct cttgatccgg caaacaaacc accgctggta gcggtggttt ttttgtttgc 660

aagcagcaga ttacgcgcag aaaaaaagga tctcaagaag atcctttgat cttttctacg 720

gggtctgacg ctcagtggaa cgaaaactca cgttaaggga ttttggtcat gagattatca 780

aaaaggatct tcacctagat ccttttaaat taaaaatgaa gttttaaatc aatctaaagt 840

atatatgagt aaacttggtc tgacagttac caatgcttaa tcagtgaggc acctatctca 900

gcgatctgtc tatttcgttc atccatagtt gcctgactcc ccgtcgtgta gataactacg 960

atacgggagg gcttaccatc tggccccagt gctgcaatga taccgcgaga cccacgctca 1020

ccggctccag atttatcagc aataaaccag ccagccggaa gggccgagcg cagaagtggt 1080

cctgcaactt tatccgcctc catccagtct attaattgtt gccgggaagc tagagtaagt 1140

agttcgccag ttaatagttt gcgcaacgtt gttgccattg ctacaggcat cgtggtgtca 1200

cgctcgtcgt ttggtatggc ttcattcagc tccggttccc aacgatcaag gcgagttaca 1260

tgatccccca tgttgtgcaa aaaagcggtt agctccttcg gtcctccgat cgttgtcaga 1320

agtaagttgg ccgcagtgtt atcactcatg gttatggcag cactgcataa ttctcttact 1380

gtcatgccat ccgtaagatg cttttctgtg actggtgagt actcaaccaa gtcattctga 1440

gaatagtgta tgcggcgacc gagttgctct tgcccggcgt caatacggga taataccgcg 1500

ccacatagca gaactttaaa agtgctcatc attggaaaac gttcttcggg gcgaaaactc 1560

tcaaggatct taccgctgtt gagatccagt tcgatgtaac ccactcgtgc acccaactga 1620

tcttcagcat cttttacttt caccagcgtt tctgggtgag caaaaacagg aaggcaaaat 1680

gccgcaaaaa agggaataag ggcgacacgg aaatgttgaa tactcatact cttccttttt 1740

caatattatt gaagcattta tcagggttat tgtctcatga gcggatacat atttgaatgt 1800

atttagaaaa ataaacaaat aggggttccg cgcacatttc cccgaaaagt gccacctgac 1860

gtctaagaaa ccattattat catgacatta acctataaaa ataggcgtat cacgaggccc 1920

tttcaatgct ggaaggagct tgcctttcgg tccctgtaca tccggacatg aattgaccgt 1980

tgcagaccag ctaatttgca ttccatcgct cgtctcgagg tccacacttg aaggttcttg 2040

gagccccgac acgtttgaac agatacacta gtcatgtaaa tcatcttgtt gtcttaaact 2100

gactagtcgg tggctgagaa tgacagcatc aggcaagatg gcttcctatc caagggaacg 2160

caatttgaac actagaggag aacagtggcc ccggaatctc gactgagaag ctgctgctct 2220

tccagcaaac actcgtgaaa gcatggtagc ccatatgttg tcggggatcg gcaacactag 2280

ttaccgaaca gagtcccgct ggtgctttga agtagatatt agtattcccc tgccttggat 2340

tgaatcttcc tacttcctag acacgctata ttggtatccc agataattta atctattgtt 2400

aggagggtat ctttgaatat tttctccgga aaaaacccga ccaaccagtg cctatggcat 2460

attattaatt aattaatcca gtggttgata ttggaggaga tttagtgcca cgaatggttg 2520

cctagcgcgt ggtggaagga ggataatatg gaagcttggc atttgcatcc tgtgcagaaa 2580

ctgccagcta acgaacccct ggttaatgtg gactagcaac gtgatccgct tgtttcaggg 2640

tgagaactac ggagatgggt tcattctcgt tttggtgggc ttggaagatt ctatgtatcc 2700

gcctcggatg cctcgggagg ccgagtcttt agggcagtag ggctagggat cgtcgagatg 2760

ccaatgctgc cgatcatgag ggtgttcccg agaaccatat gtcaagtgtt cattgctctc 2820

tttttctcac cagccgagga agaagaagcc ggcaaataac gcaactgccg agaatttacc 2880

atcagcgata ataacgcgat cgataagaaa agaagcgaaa gcataataaa ggaatgtctc 2940

gggatggcgc cgaaagtagc aaaaacccgg atgcctaaag gttcccgagt ggggaaggcg 3000

cgctggccct actgggcgct actgcccttt tgggatccat cggaccctgt cttttcttcg 3060

agcttagcga gcttggctgg ccttccttcc tgttccgtgc aagtctagat cccacttcat 3120

ttcatcctca aaccgccgtc tccatccagt cccttcgatc gttcgatgtg acttccaggg 3180

tcctgatccc attctcatac ttccgatgtc tcgccgtaat atccaagaaa aggagaagag 3240

tggaggacac aacaagtcaa atgaaaaacc tacttataga acctttgtat ccgtctccat 3300

gggttctctt ccattccctc atcacgctct cagtctctct ttcttgtctt tcctttccca 3360

gcttctctct agtccttcct taatcgtacc cggttgatcc aggttccatt ccttcattat 3420

catcacgggt ctctggccag ctgtgttgag ccttcctagc aaggctgcgt gaggttctcg 3480

ttcaggactc tggcatcacg ccgggagtgt ttgtattagc ttttaatttt agtgtatcat 3540

tggaaaaatg catgactcga gtctaattta cactgatcaa caaaccaagt acagtgatca 3600

atacatcaag tatctggaag cgaatctaga agacaaccga cccattaatg tttggatata 3660

ccgccattta aaagtgaggg cgtttccatc tggactgacc aggcgtggct ctctcatcga 3720

gtgccacgca agcaggtctg ctcactgtga tcacatctgt cttacacccg ctgtagtaca 3780

aactattctc ccgacgcgta tctagcacat ctttacttta tcgataggca catgttcttc 3840

cattcaataa gcagacaagc ccattccaat ctcaagccac gtccgcctcc gatcgcacac 3900

taatcttccg ccgccaaact caaagagctc tgtacagtga ccggtgactc tttctggcat 3960

gcggagagac ggacggtcgc agagaggagg gctgagtaat aagcgcactc atgtcagctc 4020

tggcgctctg aggtgcagtg gatgattatt aatccgggac cggccgcccc tccgccccga 4080

agtggaaagg ctggtgtgcc cctcgttgac caagaatcta ttgcatcatc ggagaatatg 4140

gagcttcatc gaatcaccgg cagtaagcga aggagaatgt gaagccaggg gtgtatagcc 4200

gtcggcgaaa tagcatgcca ttaacctagg tacagaagtc caattgcttc cgatctggta 4260

aaagattcac gagatagtac cttctccgaa gtaggtagag cgagtacccg gcgcgtaagc 4320

tccctaattg gcccatccgg catctgtagg gcgtccaaat atcgtgcctc tcctgctttg 4380

cccggtgtat gaaaccggaa aggccgctca ggagctggcc agcggcgcag accgggaaca 4440

caagctggca gtcgacccat ccggtgctct gcactcgacc tgctgaggtc cctcagtccc 4500

tggtaggcag ctttgccccg tctgtccgcc cggtgtgtcg gcggggttga caaggtcgtt 4560

gcgtcagtcc aacatttgtt gccatatttt cctgctctcc ccaccagctg ctcttttctt 4620

ttctctttct tttcccatct tcagtatatt catcttccca tccaagaacc tttatttccc 4680

ctaagtaagt actttgctac atccatactc catccttccc atcccttatt cctttgaacc 4740

tttcagttcg agctttccca cttcatcgca gcttgactaa cagctacccc gcttgagcag 4800

acatcacaaa tgcctcaatc ctgggaagaa ctggccgctg ataagcgcgc ccgcctcgca 4860

aaaaccatcc ctgatgaatg gaaagtccag acgctgcctg cggaagacag cgttattgat 4920

ttcccaaaga aatcggggat cctttcagag gccgaactga agatcacaga ggcctccgct 4980

gcagatcttg tgtccaagct ggcggccgga gagttgacct cggtggaagt tacgctagca 5040

ttctgtaaac gggcagcaat cgcccagcag ttaacaaact gcgcccacga gttcttccct 5100

gacgccgctc tcgcgcaggc aagggaactc gatgaatact acgcaaagca caagcgcccc 5160

gttggtccac tccatggcct ccccatctct ctcaaagacc agcttcgagt caagggctac 5220

gaaacatcaa tgggctacat ctcatggcta aacaagtacg acgaagggga ctcggttctg 5280

acaaccatgc tccgcaaagc cggtgccgtc ttctacgtca agacctctgt cccgcagacc 5340

ctgatggtct gcgagacagt caacaacatc atcgggcgca ccgtcaaccc acgcaacaag 5400

aactggtcgt gcggcggcag ttctggtggt gagggtgcga tcgttgggat tcgtggtggc 5460

gtcatcggtg taggaacgga tatcggtggc tcgattcgag tgccggccgc gttcaacttc 5520

ctgtacggtc taaggccgag tcatgggcgg ctgccgtatg caaagatggc gaacagcatg 5580

gagggtcagg agacggtgca cagcgttgtc gggccgatta cgcactctgt tgaggacctc 5640

cgcctcttca ccaaatccgt cctcggtcag gagccatgga aatacgactc caaggtcatc 5700

cccatgccct ggcgccagtc cgagtcggac attattgcct ccaagatcaa gaacggcggg 5760

ctcaatatcg gctactacaa cttcgacggc aatgtccttc cacaccctcc tatcctgcgc 5820

ggcgtggaaa ccaccgtcgc cgcactcgcc aaagccggtc acaccgtgac cccgtggacg 5880

ccatacaagc acgatttcgg ccacgatctc atctcccata tctacgcggc tgacggcagc 5940

gccgacgtaa tgcgcgatat cagtgcatcc ggcgagccgg cgattccaaa tatcaaagac 6000

ctactgaacc cgaacatcaa agctgttaac atgaacgagc tctgggacac gcatctccag 6060

aagtggaatt accagatgga gtaccttgag aaatggcggg aggctgaaga aaaggccggg 6120

aaggaactgg acgccatcat cgcgccgatt acgcctaccg ctgcggtacg gcatgaccag 6180

ttccggtact atgggtatgc ctctgtgatc aacctgctgg atttcacgag cgtggttgtt 6240

ccggttacct ttgcggataa gaacatcgat aagaagaatg agagtttcaa ggcggttagt 6300

gagcttgatg ccctcgtgca ggaagagtat gatccggagg cgtaccatgg ggcaccggtt 6360

gcagtgcagg ttatcggacg gagactcagt gaagagagga cgttggcgat tgcagaggaa 6420

gtggggaagt tgctgggaaa tgtggtgact ccataggctt tgatttaata gctccatgtc 6480

aacaagaata aaacgcgttt cgggtttacc tcttccagat acagctcatc tgcaatgcat 6540

taatgcattg gacctcgcaa ccctagtacg cccttcaggc tccggcgaag cagaagaata 6600

gcttagcaga gtctattttc attttcggga gacgagatca agcagatcaa cggtcgtcaa 6660

gagacttacg agactgagga atccgctctt ggctccacgc gactatatat ttgtctctaa 6720

ttgtactttg acatgctcct cttctttact ctgatagctt gactatgaaa attccgtcac 6780

cagcccctgg gttcgcaaag ataattgcac tgtttcttcc ttgaactctc aagcctacag 6840

gacacacatt catcgtaggt acgggtctct ggccagctgt gttgagcctt cctagcaagg 6900

ctgcgtgagg ttctcgttca ggactctggc atcacgccgg gagtgtttgt attagctttt 6960

aattttagtg tatcattgga aaaatgcatg actcgagtct aatttacact gatcaacaaa 7020

ccaagtacag tgatcaatac atcaagtatc tggaagcgaa tctagaagac aaccgaccca 7080

ttaatgtttg gatataccgc catttaaaag tgagggcgtt tccatctgga ctgaccaggc 7140

gtggctctct catcgagtgc cacgcaagca ggtctgctca ctgtgatcac atctgtctta 7200

cacccgctgt agtacaaact attctcccga cgcgtatcta gcacatcttt actttatcga 7260

taggcacatg ttcttccatt caataagcag acaagcccat tccaatctca agccacgtcc 7320

gcctccgatc gcacactaat cttccgccgc caaactcaaa agtcaaaaca aagcctccac 7380

ctgcgtctgc gtgatggggg aaaaaaaagg agcggtgatg tagtcaggat gaattgcatt 7440

gtgcatcttg gactaagacg gagtgggtag gggatcggca agcgaccgtt acaccaagat 7500

gtgatgccag ccaggtacag agagggaggc acatatcaca ttgtttgcta tcatgcaatc 7560

tatgggctta tattggaaga tagggatgga agatgtgact ggaggaggtt ggtaggtagg 7620

ttagtactca tttagaaatc tattggggag atagtatctc tttctcatca catgcacaga 7680

agaggagagg aggggggaat aagcaggtcc ccgatgacat ggcggggctg aaagccttgt 7740

aactcacgat gtattaagta tttactactg ctggtagaaa agataaacct ggtctagttt 7800

attatgagtg actggaattc gaagggaggg aaactttggc cgagtcgtag aaagagagag 7860

gggtcctttc agcctgatgg acgattttcc tttggatgct gttcagctgg caagactagt 7920

catttaatag caggagctga taagaatcct gtccagggtc tggaggagat gccaccaatc 7980

actaaccatg accatcggtg tctggagact gactggttcc agtccatcgc taatagttcc 8040

cccatgcttg cttttttccc gcccacaagg cgttcgcttc agcacgaacg gcgttctcag 8100

attccttctt ctccaggtgg acccccacgg cgatccaccc gctcaacaaa ctaatggttt 8160

aacttagtct acttatcgag atagttagtt catcgtggct cagtcagctt atgatcgttg 8220

cgaacgggcg aagacggcgt cctcgggctt ggaggtgaat gtggcgacct gcgcgcgggc 8280

ttcggggacc tgaaccaaca agactctcat cttgggtccc taagtgtggc agcgaagcaa 8340

cacgagcatc acggagaagt t 8361

<210> 21
<211> 6254
<212> DNA
<213> Artificial Sequence

<220>
<223> Plasmid pBZ0061

<400> 21
gcggtaatac ggttatccac agaatcaggg gataacgcag gaaagaacat gtgagcaaaa 60

ggccagcaaa aggccaggaa ccgtaaaaag gccgcgttgc tggcgttttt ccataggctc 120

cgcccccctg acgagcatca caaaaatcga cgctcaagtc agaggtggcg aaacccgaca 180

ggactataaa gataccaggc gtttccccct ggaagctccc tcgtgcgctc tcctgttccg 240

accctgccgc ttaccggata cctgtccgcc tttctccctt cgggaagcgt ggcgctttct 300

catagctcac gctgtaggta tctcagttcg gtgtaggtcg ttcgctccaa gctgggctgt 360

gtgcacgaac cccccgttca gcccgaccgc tgcgccttat ccggtaacta tcgtcttgag 420

tccaacccgg taagacacga cttatcgcca ctggcagcag ccactggtaa caggattagc 480

agagcgaggt atgtaggcgg tgctacagag ttcttgaagt ggtggcctaa ctacggctac 540

actagaagaa cagtatttgg tatctgcgct ctgctgaagc cagttacctt cggaaaaaga 600

gttggtagct cttgatccgg caaacaaacc accgctggta gcggtggttt ttttgtttgc 660

aagcagcaga ttacgcgcag aaaaaaagga tctcaagaag atcctttgat cttttctacg 720

gggtctgacg ctcagtggaa cgaaaactca cgttaaggga ttttggtcat gagattatca 780

aaaaggatct tcacctagat ccttttaaat taaaaatgaa gttttaaatc aatctaaagt 840

atatatgagt aaacttggtc tgacagttac caatgcttaa tcagtgaggc acctatctca 900

gcgatctgtc tatttcgttc atccatagtt gcctgactcc ccgtcgtgta gataactacg 960

atacgggagg gcttaccatc tggccccagt gctgcaatga taccgcgaga cccacgctca 1020

ccggctccag atttatcagc aataaaccag ccagccggaa gggccgagcg cagaagtggt 1080

cctgcaactt tatccgcctc catccagtct attaattgtt gccgggaagc tagagtaagt 1140

agttcgccag ttaatagttt gcgcaacgtt gttgccattg ctacaggcat cgtggtgtca 1200

cgctcgtcgt ttggtatggc ttcattcagc tccggttccc aacgatcaag gcgagttaca 1260

tgatccccca tgttgtgcaa aaaagcggtt agctccttcg gtcctccgat cgttgtcaga 1320

agtaagttgg ccgcagtgtt atcactcatg gttatggcag cactgcataa ttctcttact 1380

gtcatgccat ccgtaagatg cttttctgtg actggtgagt actcaaccaa gtcattctga 1440

gaatagtgta tgcggcgacc gagttgctct tgcccggcgt caatacggga taataccgcg 1500

ccacatagca gaactttaaa agtgctcatc attggaaaac gttcttcggg gcgaaaactc 1560

tcaaggatct taccgctgtt gagatccagt tcgatgtaac ccactcgtgc acccaactga 1620

tcttcagcat cttttacttt caccagcgtt tctgggtgag caaaaacagg aaggcaaaat 1680

gccgcaaaaa agggaataag ggcgacacgg aaatgttgaa tactcatact cttccttttt 1740

caatattatt gaagcattta tcagggttat tgtctcatga gcggatacat atttgaatgt 1800

atttagaaaa ataaacaaat aggggttccg cgcacatttc cccgaaaagt gccacctgac 1860

gtctaagaaa ccattattat catgacatta acctataaaa ataggcgtat cacgaggccc 1920

tttcgatcat tgatccaccg cccacgaggc gcgtctttgc tttttgcgcg gcgtccaggt 1980

tcaactctct ctgcagctcc agtccaacgc tgactgacta gtttacctac tggtctgatc 2040

ggctccatca gagctatggc gttatcccgt gccgttgctg cgcaatcgct atcttgatcg 2100

caaccttgaa ctcactcttg ttttaatagt gatcttggtg acggagtgtc ggtgagtgac 2160

aaccaacatc gtgcaaggga gattgatacg gaattgtcgc tcccatcatg atgttcttgc 2220

cggctttgtt ggccctattc gtgggatgcg atgccctcgc tgtgcagcag caggtactgc 2280

tggatgagga gccatcggtc tctgcacgca aacccaactt cctcttcatt ctcacggatg 2340

atcaggatct ccggatgaat tctccggcgt atatgccgta tacgcaggcg agaatcaagg 2400

aaaagggtac cgagttcttg aaccatttcg tcactaccgc gctttgctgt ccgtcgcgcg 2460

tgagtctttg gacgggaaga caggctcata atactaatgt gacggatgtg aacccgcctt 2520

atggtatgga cactgcttcg atcggtcttg attcttcagc gtggttacaa ttgctaatgc 2580

ggcataggcg gataccccaa attcgtcgct caaggcttca acgaaaactt cctccccgtt 2640

tggctgcagt ccgccggtta caatacctac tacacgggga agctgttcaa ctcgcacagt 2700

gtcgctacct ataacgcgcc ctttgtgaac ggtttcaatg gctccgactt cctcctcgac 2760

ccccacacat attcctactg gaatgcgaca taccagcgaa accatgagcc tccgcggagt 2820

tacgagggac aatatactac ggatgtgatg aaggagaagg catcgggatt gttggcagat 2880

gcgctggaca gtgacgcgcc attcttcctg acggtcgcgc cgatcgcacc gcacacgaac 2940

atcgatgtgg aggggctgag cggtgcgggt ggaccgaaga tgacagagcc gctgcctgca 3000

ccgagacatg cgcatttgtt tgctgatgca aaggtgccgc ggacgcctaa tttcaatccg 3060

gacaaggtgt gtgatatcct gacacagtgg tggggacggg cactgacaag agtaggattc 3120

tggtgcgggg tggatccaaa ccatggaact acagaaccag accgtcatcg actacgaaga 3180

ccatctttat cgccagcgtc tgcgcacttt gcaagccgtc gatgagatgg tggatgcgct 3240

gatcacgcag ctggaagaaa gtgggcagat cgacaatacc tacatcattt acagtgctga 3300

taacggctac cacattggcc atcaccgtct accccccggc aagacaactg gctatgaaga 3360

ggacattcgc gtaccattct acattcgcgg acctggcatt cctgagggaa agagcgttga 3420

ccgtcctctc gtatgcagag gaaatctccc ctgatcttcc gaactggtcg tacctggcga 3480

cctatgacta tggcacccca gttctgggga ccttccacgg aagtgacctg ctgcaggtgt 3540

tctatgggat caagccaaac tatgcagcta gttctagcca cacgtactat ctgagctttg 3600

tgtatacgct ggatccgaac tccaaccggg gggagtacat tgagtggccg cagtggaagg 3660

aatcgcggca gttgatgaat ttcggagcga acgacgccag tctccttacg gatgatttcc 3720

gcaacgggac atatgagttc atcctgcaga ataccgcggc gttccacatc tgatgccatt 3780

ggcggagggg tccggacggt caggaactta gccttatgag atgaatgatg gacgtgtctg 3840

gcctcggaaa aggatatatg gggatcatga tagtactagc catattaatg aagggcatat 3900

accacgcgtt ggacctgcgt tatagcttcc cgttagttat agtaccatcg ttataccagc 3960

caatcaagtc accacgcacg accggggacg gcgaatcccc gggaattgaa agaaattgca 4020

tcccaggcca gtgaggccag cgattggcca cctctccaag gcacagggcc attctgcagc 4080

gctggtggat tcatcgcaat ttcccccggc ccggcccgac accgctatag gctggttctc 4140

ccacaccatc ggagattcgt cgcctaatgt ctcgtccgtt cacaagctga agagcttgaa 4200

gtggcgagat gtctctgcag gaattcaagc tagatgctaa gcgatattgc atggcaatat 4260

gtgttgatgc atgtgcttct tccttcagct tcccctcgtg cagatgaggt ttggctataa 4320

attgaagtgg ttggtcgggg ttccgtgagg ggctgaagtg cttcctccct tttagacgca 4380

actgagagcc tgagcttcat ccccagcatc attacacctc agcaatgtcg ttccgatctc 4440

tactcgccct gagcggcctc gtctgcacag ggttggcaaa tgtgatttcc aagcgcggct 4500

ctattggagc ggcctccatg gaattctgct tcgacgtgtt caaggagttg aaggtccacc 4560

acgctaacga aaatattttc tactgtccga ttgctattat gtccgccctc gcgatggtat 4620

acctgggcgc caaggactct acccgaactc agatcaataa ggtcgtgaga ttcgataagc 4680

tgcctggatt cggtgattcg atcgaagcgc agtgcggcac ctccgtgaat gtccacagca 4740

gtctgcgaga tattctgaac caaatcacta agcccaacga tgtgtatagt ttcagcctgg 4800

cctctcgtct ctacgcagaa gagcgctacc ctattctgcc cgaatacttg cagtgcgtga 4860

aggaactcta ccgagggggc ctggagccca taaacttcca gacggctgct gaccaagccc 4920

gggagctcat caacagttgg gttgagtcac agactaacgg tatcattcgc aacgtgctcc 4980

agcctagcag tgttgacagc cagaccgcga tggtgctagt caacgccatt gtgttcaagg 5040

gtctctggga gaaggccttt aaggacgagg atacacaggc tatgcccttc cgcgtgaccg 5100

aacaggaaag caagcccgtc cagatgatgt accagattgg ccttttccgt gtcgcaagca 5160

tggcgagcga aaagatgaag atcctggagc tcccgtttgc ctcgggtacc atgtccatgc 5220

tcgttctcct tccggacgaa gtgagcggac tggagcagct ggagagcatc atcaacttcg 5280

agaagttgac ggagtggact agctctaacg ttatggagga gcgcaagatt aaggtgtatc 5340

tcccccggat gaagatggag gagaagtaca acctgacctc tgtgctgatg gctatgggca 5400

ttaccgatgt gtttagttcc tccgctaact tgtccgggat ctcgtcggct gagtcactca 5460

agatttccca agctgtccac gctgctcacg ctgagatcaa cgaggctggt cgtgaagtcg 5520

tcggctcggc agaagctggc gtcgatgcgg catctgtctc tgaggagttc cgcgccgacc 5580

acccgtttct attctgcatc aagcacatcg caaccaatgc cgttctgttc ttcggcaggt 5640

gtgtgtcgcc gtagacaatc aatccatttc gctatagtta aaggatgggg atgagggcaa 5700

ttggttatat gatcatgtat gtagtgggtg tgcataatag tagtgaaatg gaagccaagt 5760

catgtgattg taatcgaccg acggaattga ggatatccgg aaatacagac accgtgaaag 5820

ccatggtctt tccttcgtgt agaagaccag acagacagtc cctgatttac ccttgcacaa 5880

agcactagaa aattagcatt ccatccttct ctgcttgctc tgctgatatc actgtcattc 5940

aatgcatagc catgagctca tcttagatcc aagcacgtaa ttccatagcc gaggtccaca 6000

gtggagcagc aacattcccc atcattgctt tccccagggg cctcccaacg actaaatcaa 6060

gagtatatct ctaccgtcca atagatcgtc ttcgcttcaa aatctttgac aattccaaga 6120

gggtccccat ccatcaaacc cagttcaata atagccgaga tgcatggtgg agtcaattag 6180

gcagtattgc tggaatgtcg gggccagttg gcccggtggt cattggccgc ctgtgatgcc 6240

atctgccact aaat 6254